# EUROPEAN PATENT SPECIFICATION

(11) **EP 1 140 162 B1**
(45) Date of publication and mention of the grant of the patent: **25.08.2004**
(21) Application number: 00905548.4
(22) Date of filing: 06.01.2000
(51) Int. Cl.: A61K 39/21, A61K 39/29, A61K 39/12, A61K 39/39, A61P 31/18

(54) **VACCINATION METHOD FOR EFFICIENT INDUCTION OF CYTOTOXIC T LYMPHOCYTE RESPONSE**
IMPFUNGSMETHODE ZUR EFFIZIENTEN INDUKTION DER T-LYMPHOZYTEN ZYTOTOXISCHANTWORT
METHODE DE VACCINATION VISANT A L'INDUCTION EFFICACE D'UNE REPONSE AUX LYMPHOCYTES T CYTOTOXIQUES

(30) Priority: 08.01.1999 US 115405 P
(43) Date of publication of application: 10.10.2001
(73) Proprietor: WISCONSIN ALUMNI RESEARCH FOUNDATION, Madison, WI 53705 (US); Powderject Vaccines, Inc., Madison, Wisconsin 53711 (US)
(72) Inventor: WATKINS, David, I., Arena, WI 53503 (US); ALLEN, Todd, M., Madison, WI 53705 (US); VOGEL, Thorsten, U., Madison, WI 53713 (US); FULLER, Deborah, L., Oregon, WI 53575 (US); FULLER, James, T., Oregon, WI 53575 (US)
(74) Representative: Duckworth, Timothy John
(86) International application number: PCT/US2000/000286
(87) International publication number: WO 2000/040261

(56) References cited:
- EP-A- 0 421 635
- T. HANKE ET AL.: "Enhancement of MHC class I restricted peptide-specific T cell induction by a DNA prime/MVA boost vaccination regime" VACCINE, vol. 16, no. 4, 1998, pages 439-445, XP004106957
- T. ALLEN AT AL.: "Characterisation of the peptide-binding motif of a rhesus MHC class I molecule (Mamu A*01) that binds an immunodominanat CTL epotope from simian immunodeficiency virus" J. IMMUNNOL., vol. 160, 1998, pages 6062-6071, XP002145008 cited in the application
- A. KUHRÖBER ET AL: "DNA vaccination with plasmids encoding intracellular (HBcAg) or secreted (HBeAg) forms of the core protein of hepatitis B virus primes T cell responses to two overlapping Kb and Kd-restricted epotopes" INTERNATIONAL IMMUNOLOGY, vol. 9, no. 8, 1997, pages 1203-1212, XP000929987
- T. HANKE ET AL.: "DNA multi-CTL epitope vaccines for HIV and Plasmodium falciparum: immunogenicity in mice" VACCINE, vol. 16, no. 4, 1998, pages 426-435, XP004099305

## Description

This invention was made with United States government support awarded by the following agencies:
NIH Grant No: A141913

The United States has certain rights in this invention.

### BACKGROUND TO THE INVENTION

Considerable effort has been directed toward the development of vaccines and other pharmaceuticals for preventing or controlling infections with the lentivirus human immunodeficiency virus (HIV), the causal agent of acquired immune deficiency syndrome (AIDS) in humans.

Although treatment of HIV-infected individuals with antiretroviral agents has been successful in reducing viral loads, these drugs have had little impact on the global epidemic. Reservoirs of HIV persist; in 1998 alone, there were over 5.8 million new cases of HIV infection worldwide. Almost 90% of those infected with HIV live in poverty, and are unable to afford the costly drugs used to treat HIV infections. Therefore, the development of vaccines designed to prevent HIV infections is the best hope for containing this disease.

Preliminary research directed toward developing a vaccine against HIV is generally conducted using the rhesus macaque, a primate susceptible to infection by simian immunodeficiency virus (SIV), an HIV-like lentivirus that causes immunodeficiency in rhesus macaques. The rhesus macaque is the best animal model in which to study HIV vaccines because the rhesus immune system is very similar to that of humans (e.g., they have very similar major histocompatibility complex (MHC) and T-cell receptor(TCR) genes) (Watkins, Critical Rev. Immunol. 15:1-29, 1995; Bontrop, et al., Immunol. Rev. 143:33-62, 1995). (All publications cited within the specification are incorporated by reference as if fully set forth below.)

The most successful vaccines tested to date are live attenuated SIV isolates. Unfortunately, these live attenuated vaccines have been shown to retain some virulence, which limits the potential for their widespread use (Baba, et al., Science 267:1820-1825, 1995).

There is growing evidence that cytotoxic T lymphocytes (CTL) may play a critical role in controlling lentivirus infections. Strong CTL responses are correlated with reduced plasma RNA viral loads in HIV-infected individuals (Ogg, et al., Science 279:2103-2106, 1998). Furthermore, CTL exert selective pressure on the AIDS virus, as evidenced by the eventual predominance of viral particles with mutations in those regions of the virus to which CTL responses are directed (Borrow, et al., Nature Medicine 3:205-211, 1998; Price, et al., Proc. Natl. Acad. Sci. (USA) 92:1890-1895, 1997; Koenig, et al., Nature Medicine 1:330-336, 1995; Haas, et al., J. Immunol. 157:4212-4221, 1996; Wolinsky, et al., Science 272:537-542, 1996; Evans, et al., Nature Medicine 5:1270-1276, 1999). Epidemiological studies show that the occurrence of such mutants, known as CTL escape mutants, in people infected with HIV is correlated with progression to AIDS in these individuals (Jin, et al., J. Experimental Med. 189:991-998, 1999), further indicating a role for CTL in the containment of HIV late in infection.

In a recent study, adoptive transfer of autologous HIV Gag-specific CD8⁺ CTL clones to three seropositive patients resulted in a rapid decrease in the percentage of productively infected CD4⁺T cells (Brodie, et al., Nat. Med. 5:341, 1998). Similarly, SIV-infected macaques depleted of CD8⁺ T lymphocytes through the administration of monoclonal antibodies were subsequently unable to control virus replication (Schmitz, et al., Science 283:857-860, 1999; Jin, et al., J. Experiment. Med. 189:991-998, 1999; Matano, et al., J. Virol. 72:164-169, 1998) These studies provide evidence that CD8⁺T cells play a role in controlling AIDS virus infections.

Although it has been shown that CTL are important in controlling lentivirus infections, there are no reports that vaccine-induced CTL can protect against lentivirus infection. The development and testing of vaccines capable of specific CTL induction may be important in establishing a vaccination regimen effective against HIV.

Epitope-based vaccines offer several potential advantages over vaccines that encode whole protein antigens. The epitope-based vaccines are capable of inducing a more potent response than whole protein vaccines (Ishioka, et al., J. Immunol. 162:3915-3925, 1999), and they confer.the capacity to control qualitative aspects of the immune response by simultaneously targeting multiple dominant and subdominant epitopes (Oukka, et al., J. Immunol. 157:3039-3045, 1996; Tourdot, et al., J. Immunol. 159:2391-2398, 1997). This may be particularly important to the development of HIV vaccines, because a broader immune response is likely to be crucial for controlling rapidly mutating pathogens such as HIV and hepatitis C virus (HCV) (McMichael, et al., Ann. Rev. Immunol. 15:271-296, 1997; Couillin, et al., J. Exper. Med. 18000:1129-1134, 1994; Cooper, et al., Immunity 10:439-449, 1999; Missale, et al., J. Clin. Invest. 98:706-714). In addition, the use of epitopes may overcome safety concerns associated with whole protein antigens (e.g., the expression of human papillomavirus (HPV) E6 and E7 antigens is associated with cervical carcinoma).

DNA vaccines are receiving considerable attention for their ability to induce cellular immune responses. These vaccines have been shown to induce immune responses to and, in some cases, even protect against, various pathogens such as influenza and malaria (Lodmell, et al., Nat. Med. 4:949-952, 1998; Wang, et al., Science 282:476-480, 1998; Ulmer, et al., Science 259:1745-1749, 1993; Donnelly, et al., Nat. Med. 1:583-587, 1995). Although DNA vaccines encoding whole protein have effectively induced immunodeficiency virus-specific cellular immune responses in non-human primates (Fuller, et al., J. Med. Primatol. 25:236-241, 1996; Boyer, et al., J. Med. Primatol. 25:242-250, 1996; Shiver, et al., Vaccine 15:884-887, 1997; Boyer, et al., Nat. Med. 3:526-532, 1997; Yasutomi, et al., J. Virol. 70:678-681, 1996), immunization with these vaccines does not reliably protect against vigorous AIDS-virus challenges. This lack of protection may have been due to the inability of previous DNA vaccine regimens to induce a sufficiently potent cellular immune response against HIV and SIV.

Current approaches to vaccine design have begun to take advantage of the synergistic effects of combining DNA vaccines with other approaches to induce stronger and more persistent cellular immune responses (Kent, et al., J. Virol. 72:10180-10188, 1998; Sedegah, et al., Proc. Natl. Acad. Sci. USA 95:7648-7653; Hanke, et al., Vaccine 16:439-445, 1998; Hanke, et al., Immunol. Lett. 66:177-181, 1999; Robinson, et al., Nat. Med. 5:526-543, 1999). Recently, live pox viruses, such as modified vaccinia virus Ankara (MVA), have proven to be effective vaccine vectors for inducing strong cellular immune responses (Sutler, et al., Vaccine 12:1032-1040, 1994; Hirsch, et al., J. Virol. 70:3741-3452, 1996; Set, et al., Proc. Natl. Acad. Sci. USA 95:1011-1026, 1998; Belyakov, et al., J. Virol. 72:8264-8272, 1998; Hanke, *et al*., J. Gen. Virol. 79:83-90, 1998). MVA is particularly attractive for use in humans because it maintains the ability to produce large quantities of recombinant product, yet is unable to generate infectious particles in mammalian cells due to a block in viral replication during virion assembly (Carroll, *et al*., Vaccine 15: 387-394, 1997).

A vaccination regimen that combines a DNA prime with an MVA boost generated strong protective CTL responses against malaria in mice (Hanke, *et al*., Vaccine 16:439-445, 1998). These murine studies demonstrated that the combination of DNA and MVA induces a significantly higher epitope-specific immune response in mice than DNA or MVA alone.

There remains a need for improved vaccination methods for inducing a high CTL response specific for a viral epitope in a primate susceptible to infection by a virus.

### BRIEF SUMMARY OF THE INVENTION

One aspect of the present invention is a combination comprising (a) a polynucleotide vaccine, comprising both (1) a polynucleotide sequence encoding a viral polyepitope and a major histocompatibility complex class I-restricted peptide epitope and (2) a polynucleotide sequence encoding a hepatitis B core antigen and a major histocompatibility complex class I-restricted peptide epitope, the encoding sequences being operably connected to a promoter functional in at least a portion of recipient cells, in an amount sufficient to induce in a primate a cytotoxic T lymphocyte response specific for the major histocompatibility complex class I-restricted peptide epitope; and (b) a live virus vector comprising a polynucleotide coding sequence encoding a viral polyepitope and the major histocompatibility complex class I-restricted peptide epitope of (a) operably connected to a promoter functional in at least a portion of recipient cells, in an amount sufficient to boost the specific cytotoxic T lymphocyte response induced by the polynucleotide vaccine (a), for use in a method of inducing an epitope-specific cytotoxic T lymphocyte immune response in a primate comprising the steps of delivering into cells of the primate the polynucleotide vaccine (a) and subsequently delivering the viral vector (b).

The invention also provides for the use of a polynucleotide vaccine (a) and a live viral vector (b) in the manufacture of a medicament for use in a method of inducing an epitope-specific cytotoxic T lymphocyte immune response in a primate comprising the steps of delivering the polynucleotide vaccine (a) into cells of the primate and subsequently delivering the live vaccine (b), wherein the polynucleotide vaccine (a) comprises both (1) a polynucleotide sequence encoding a viral polyepitope and a major histocompatibility complex class I-restricted peptide epitope and (2) a polynucleotide sequence encoding a hepatitis B core antigen and a major histocompatibility complex class I-restricted peptide epitope, the encoding sequences being operably connected to a promoter functional in at least a portion of recipient cells, in an amount sufficient to induce in a primate a cytotoxic T lymphocyte response specific for the major histocompatibility complex class I-restricted peptide epitope; and the live virus vector (b) comprises a polynucleotide coding sequence encoding a viral polyepitope and the major histocompatibility complex class I-restricted peptide epitope of (a) operably connected to a promoter functional in at least a portion of recipient cells, in an amount sufficient to boost the specific cytotoxic T lymphocyte response induced by the polynucleotide vaccine of (a).

The invention further provides for the use of a polynucleotide vaccine, comprising: both (1) a polynucleotide sequence encoding a viral polyepitope and a major histocompatibility complex class I-restricted peptide epitope and (2) a polynucleotide sequence encoding encoding a hepatitis B core antigen and a major histocompatibility complex class I-restricted peptide epitope, the encoding sequences being operably connected to a promoter functional in at least a portion of recipient cells, in an amount sufficient to induce in a primate a cytotoxic T lymphocyte response specific for the major histocompatibility complex class I-restricted peptide epitope; in the manufacture of a medicament for use in a method of inducing an epitope-specific cytotoxic T lymphocyte immune response in a primate comprising the steps of: (a) delivering into cells of the primate the polynucleotide vaccine; and (b) following step (a), delivering a live virus vector comprising a polynucleotide coding sequence encoding a viral polyepitope and the major histocompatibility complex class I-restricted peptide epitope of (a) operably connected to a promoter functional in at least a portion of recipient cells, in an amount sufficient to boost the specific cytotoxic T lymphocyte response induced by the polynucleotide vaccine of step (a).

The invention additionally provides for the use of a live virus vector comprising a polynucleotide coding sequence encoding a viral polyepitope and a major histocompatibility complex class I-restricted peptide epitope operably connected to a promoter functional in at least a portion of recipient primate cells in the manufacture of a medicament for use in a method of inducing an epitope-specific cytotoxic T lymphocyte immune response in a primate comprising the steps of: (a) delivering into cells of the primate a polynucleotide vaccine, the vaccine comprising both (1) a polynucleotide sequence encoding a viral polyepitope and a major histocompatibility complex class I-restricted peptide epitope and (2) a polynucleotide sequence encoding a hepatitis B core antigen and a major histocompatibility complex class I-restricted peptide epitope, the encoding sequences being operably connected to a promoter functional in at least a portion of recipient cells, and (b) following step (a), delivering the live virus vector in an amount sufficient to boost the specific cytotoxic T lymphocyte response induced by the polynucleotide vaccine of step (a); wherein the major histocompatability complex class I-restricted peptide epitope of the live virus vector is the same as the major histocompatability complex class-I-restricted peptide epitope of the polynucleotide vaccine.

In one embodiment, the epitope is a viral epitope. Preferably, the viral epitope employed in the vaccine of the present invention is an HIV eptiope. Preferably, the epitope is derived from a conserved region of an HIV protein such as gag, env, or nef.

In another embodiment of the present invention, the epitope is a tumor epitope.

It is an object of the present invention to induce a viral epitope-specific CTL response in a primate so as to reduce the susceptibility of the primate to infection upon exposure to the virus.

It is an advantage that the invention induces a CTL immune response in primates comparable to that observed in acute viral infections.

It is an advantage that the invention avoids problems associated with the use of live, attenuated virus and whole protein vaccines by employing an epitope vaccine.

### BRIEF DESCRIPTION OF THE SEVERAL VIEWS OF THE DRAWINGS

Fig. 1 shows the DNA and MVA vaccination schedules for the subject macaques.
Fig. 2A and 2B show the CTL response measured by ⁵¹Cr-release assays for immunized macaques as a function of the number of vaccinations received. Fig. 2C and 2D show the CTL response measured by tetramer straining of *in vitro* cultures.
Fig. 3 shows the percentage of p11C, C->M-specific CD3⁺/CD8a⁺ T lymphocytes, as measured by tetramer staining, as a function of time.
Fig. 4 shows the frequency of IFN-γ-producing T cells in immunized macaques using the ELISPOT assay.
Fig. 5 shows the fraction of tetramer positive PBMC that produce gamma interferon when unstimulated, or stimulated with PMA/Ionomycin or p11C,C->M peptide.
Fig. 6 shows the percent specific lysis as measured by a Cr⁵¹-release assay using freshly isolated PBMC from immunized macaques.

### DETAILED DESCRIPTION OF THE INVENTION

The ability of an MVA boost to enhance CTL response to a specific epitope following a DNA prime in rhesus macaques was assessed. Although vaccination with MVA was reported to boost CTL responses in mice (Hanke, et al., Vaccine 16:439-445, 1998), the efficacy of this approach in primates was uncertain, because vaccine immunogenicity results in mice cannot generally be extrapolated to primates.

The Mamu-A*01-restricted SIV gag CTL epitope (CTPYDINQM, SEQ ID NO:1) was chosen as the epitope in vaccine studies described in the Examples because all Mamu-A*01 rhesus macaques studied to date demonstrate significant CTL responses to this epitope (Miller, et al., J. Immunol. 147:320-329, 1991). Furthermore, the optimal length of the epitope has now been defined (Allen, et al., J. Virol. 160:6062-6071, 1998). Optimal epitope length and definition is important in the design of epitope-based vaccines and in the construction of epitope-specific tetrameric complexes used in the assays described below.

As described in the Examples, Mamu-A*O1 rhesus macaques were vaccinated with naked DNA. A subgroup of DNA-vaccinated subjects received at least one boost with MVA. At least one DNA vaccine comprising a sequence encoding the Mamu-A*01-restricted CTL epitope CTPYDINQM (C->M) (Allen, et al., J. Immunol. 160:6062-6071, 1998) was given as a prime, followed by boosting with MVA containing the same CTL epitope. The DNA prime included at least one of two plasmids encoding the epitope inserted within the HIV/SIV polyepitope (HW), or within the immunodominant loop of the Hepatitis B core antigen (HBcAg) (Allen, et al., "Direct *ex vivo* staining by MHC class I tetramers in fresh PBL of rhesus macauqes DNA vaccinated with a Hepatitis B core antigen carrier vector, to be submitted).

The Hepatitis B core antigen was employed because it was shown to be effective in inducing humoral and cellular immune responses, and it can easily accommodate foreign epitopes from other pathogens (Schodel, Intervirology 39:104-110, 1996; Schodel, et al., J. Biotechnology 44:91-96, 1996). Factors responsible for its exceptional immunogenicity as a carrier moiety include its multimeric structure, which results in a high epitope density per particle, and the provision of T cell help (Ulrich, et al., Advances in Virus Research 50:141-182, 1998). More importantly, when used as a delivery vehicle of CTL epitopes, the HBcAg enhances Th₁-like responses, presumably a result of the structural form of the immunogen (Milich, et al., J. Virol. 71:2192-2201, 1997). Therefore, a DNA vaccine encoding recombinant virus-like carrier particles such as HBcAg may further enhance the CTL induction capabilities of DNA vaccines.

It was demonstrated that strong proliferative and antibody responses to the HBcAg correlate with the DNA vaccine's ability to induce CTL to the SIV epitope. The mechanism by which the HBcAg enhances CTL response has not been established, although it is likely that immune response to HBcAg plays a significant role in enhancing specific CTL responses against the heterologous SIV epitope. Therefore, inclusion of CTL epitopes within this HBcAg may represent a particularly robust method of priming CTL responses in primates.

Following vaccination with the DNA vaccine, an MVA encoding the HW polyepitope, including the SIV C->M epitope, was used to boost primary CTL responses induced by the DNA vaccinations. Three of the immunized animals were evaluated to determine the effectiveness of the immunization regimen. The immunization regimen was found to induce high levels of CTL, with up 18% of all freshly isolated, unstimulated CD8⁺ T-lymphocytes exhibiting specificity for the C->M epitope. These findings illustrate the potential of this vaccination method to induce strong CTL responses in primates.

By the DNA/MVA vaccine of the present invention, we have obtained the highest reported levels of antigen-specific CD8⁺ T lymphocyte responses detected in non-human primates. Initially, gene gun DNA vaccinations targeted to dermal and mucosal tissues proved effective at priming p11C, C->M-specific CD8⁺ T cell responses in Mamu-A*01⁺ rhesus macaques, in some cases even after a single vaccination. Tetramer staining of fresh, unstimulated PBMC revealed that some DNA-vaccinated animals had levels of epitope-specific CD3⁺/CD8α⁺ T lymphocytes as high as 0.5% after DNA vaccination only. Boosting of six of these DNA vaccinated macaques with recombinant MVA expressing the same p11C, C->M epitope dramatically increased the levels of antigen-specific responses.

Tetramer staining of fresh, unstimulated PBMC from two of the six DNA/MVA vaccinated animals indicated that levels as high as 18% and 20.0% of CD3⁺/CD8α⁺ T lymphocytes were specific for the immunizing epitope. In the other four vaccinated animals, levels between 1.2% to 8.3% were detected.

ELISPOT analysis confirmed the presence of high levels of antigen-specific cells in these animals. The induction of IFN-γ in tetramer positive T lymphocytes, as measured by intracellular IFN-γ staining, indicated that these CD8⁻ cells were functionally active. Furthermore, in five of these six DNA/MVA vaccinated animals the levels of antigen-specific T lymphocytes were sufficiently high to detect, for the first time, direct *ex vivo* vaccine-induced cytotoxic activity in non-human primates. Therefore, this epitope-based DNA/MVA vaccination regimen induced levels of antigen-specific CD8⁺ T lymphocytes equivalent to those observed in acutely SIV-infected rhesus macaques (Kuroda, et al., J. Immunol. 162:5127-5133, 1999).

The responses induced by our DNA/MVA vaccination regimen are difficult to compare with those induced by other vaccines. Prior to the advent of tetramers, estimation of precursor CTL levels was typically determined by limiting dilution analysis (LDA), assays which were not utilized in our study. However, recent studies have begun to re-examine the levels of antigen specific CD8⁺ T cells present during natural and experimental viral infections using tetramers and functional assays to measure single cell interferon-gamma (IFN-γ) production (ELISPOT) (Murali-Krishna, et al., Immunity 8:177-187, 1998; Lalvani, et al., J. Exp. Medicine 186:859-865, 1997; McMichael, et al., J. Exp. Medicine 187:1367-1371, 1998). These analyses have revealed that LDA typically underestimates the level of antigen-specific CD8⁺ T cells by 10- to 100-fold as compared with tetramer or ELISPOT analysis. Assuming a maximum underestimation, LDA values for SIV-specific CD8⁺ T lymphocytes from rhesus macaques immunized with a vaccinia virus-based subunit vaccine (Kent, et al., J. Virol. 70:4941-4947, 1996), or immune-stimulating complexes (iscoms) carrying proteins or lipopeptides (Hulskotte, et al., J. Virol. 69:6289-6296, 1995), would indicate the induction of between 100 and 2,000 antigen-specific CD8⁺ T lymphocytes per 10⁶ PBMC. In our study, tetramer staining indicated that approximately 0.5% of CD3⁺/CD8α⁺ T cells from three of our DNA vaccinated animals were antigen-specific. Conservatively assuming that 20% of rhesus PBMC are CD3⁺/CD8⁺ T cells, these tetramer values would extrapolate to 1,000 per 10⁶ PBMC (or 1/1000) being specific for the p11C, C->M epitope. Therefore, the levels of antigen-specific responses induced by the DNA vaccinations in this study were equivalent to some previously published levels of SIV-specific vaccine-induced CTL in non-human primates (Kent, et al., J. Virol. 70:4941-4947, 1996; Hulskotte, et al., J. Virol. 69:6289-6296, 1995).

Corrected LDA values from SIV-infected macaques indicate that 600 to 10,000 per 10⁶ PBMC would be specific for a given SIV protein (Kent, et al., J. Virol. 70:4941-4947, 1996; Hulskotte, et al., J. Virol. 69:6289-6296, 1995; Venet, et al., J. Immunol. 148:2899-29908, 1992). More recent tetramer analysis of p11C, C->M-specific T lymphocytes in Mamu-A*01⁺ macaques chronically infected with SIV have indicated that between 0.7% and 10.3% of CD3⁺/CD8⁺ T cells were epitope specific (Kuroda, et al., J. Exp. Medicine 187:1373-1381, 1998; Kuroda, et al., J. Immunol. 162:5127-5133, 1999). These tetramer values extrapolate to 1,000 to 20,000 per 10⁶ PBMC, values similar to the corrected LDA values. The tetramer levels detected in the DNA/MVA vaccinated animals in our study (1.2% to 20.0%) were equivalent to, and in some cases significantly greater than, levels detected in acutely SIV-infected macaques ((Kuroda, et al., J. Immunol. 162:5127-5133, 1999). Therefore, this epitope-based DNA/MVA vaccine regimen induced the highest levels of antigen-specific CD8⁺ T cell responses ever recorded in a non-human primate.

The induction of p11C, C->M-specific CD8⁺ responses in DNA/MVA vaccinated macaques has also been recently reported (Hanke, et al., Immunol. Lett. 66:177-181, 1999; Hanke, et al., "Effective induction of SIV-specific CTL in macaques using a DNA prime-MVA boost vaccination regiment," J. Virol. *In press*). In these experiments, tetramer staining of 1.3%, 4.9%, and 1.5% of CD8α⁺ (not CD3⁺/CD8α⁺) T lymphocytes in frozen PBMC were induced in three Mamu-A*01⁺ macaques following a regimen of 2 DNA and 2 MVA vaccinations. In our DNA/MVA vaccinated animals, responses following either the first or second MVA were, in some cases, substantially higher. This is likely due to differences in the vaccine regimen which in our experiments employed more DNA vaccinations, delivered greater amounts of DNA, and included a second DNA vector. In fact, no significant lytic activity had been detected in two-week *in vitro* stimulated cultures derived from PBMC of the DNA-vaccinated macaques although the CTL responses were primed (Hanke, et al., supra, 1999). The additional vector used in our experiments, which expressed the p11C, C->M epitope in the context of the hepatitis B core antigen, was included to provide T cell helper responses during the DNA vaccination. 'In some animals, responses to the hepatitis B core antigen appeared to correlate with the induction of good CTL responses. However, the overall significance of the hepatitis B core antigen in inducing these responses is still being investigated. Therefore, the more effective priming of CTL responses by our DNA vaccinations likely account for the higher levels of p11C, C->M-specific CD8⁺ cells induced by the MVA boost in our vaccinated animals.

The induction of p11C, C->M-specific responses has also recently been reported in Mamu-A*01⁺ macaques vaccinated with MVA alone expressing gag/pol of SIV (Seth, et al., Proc. Natl. Acad. Sci. USA 95:10112-10116, 1998). Tetramer staining levels ranged in these animals between 1% and 5% of CD3⁺/CD8b⁺ cells. These responses, however, were not detectable until after the second MVA administration. In both our experiments and those of Hanke, et al. (Hanke, et al., Immunol. Lett. 66:177-181, 1999; Hank, et al., "Effective induction of SIV-specific CTL in macaques using a DNA prime-MVA boost vaccination regiment," J. Virol. *in press*), a single MVA administration following the DNA priming was sufficient to induce similar, if not greater, levels of p11C, C->M-specific responses.

While MHC class I tetramers are effective at determining the levels of antigen-specific CD8⁺ T lymphocytes in fresh PBMC, the functional state of these cells is unknown. In our studies the ability to measure p11C, C->M-specific induction of IFN-γ in fresh PBMC by both ELISPOT and intracellular IFN-γ staining was critical to assessing the function of the vaccine-induced CD8⁺ T lymphocytes. Virtually all of the cells which stained positive for the tetramers were found to be capable of producing IFN-γ in response to the p11C, C->M peptide. These analyses were not undertaken in previous primate studies where high level antigen-specific CD8⁺ T cell responses were induced by MVA or DNA/MVA vaccines (Seth, et al., Proc. Natl. Acad. Sci. USA 95:10112-10116, 1998; Hanke, et al., Immunol. Lett. 66:177-181, 1999; Hank, et al., "Effective induction of SIV-specific CTL in macaques using a DNA prime-MVA boost vaccination regiment," J. Virol. *in press*). In comparing the number of p11C, C->M-specific cells detected by ELISPOT and tetramer staining, ELISPOT generally underestimated levels by between 1.3-fold and 10-fold. While this may reflect that not all tetramer positive cells produce IFN-γ, recent studies with ELISPOT (Seth, et al., Proc. Natl. Acad. Sci. USA 95:10112-10116, 1998) (and our unpublished findings) suggest that the 1 µM concentration of peptide used in these experiments may be insufficient to induce the maximal number of antigen-specific SFCs (spot-forming cells), indicating the need to properly titrate individual peptides for maximal responses in ELISPOT.

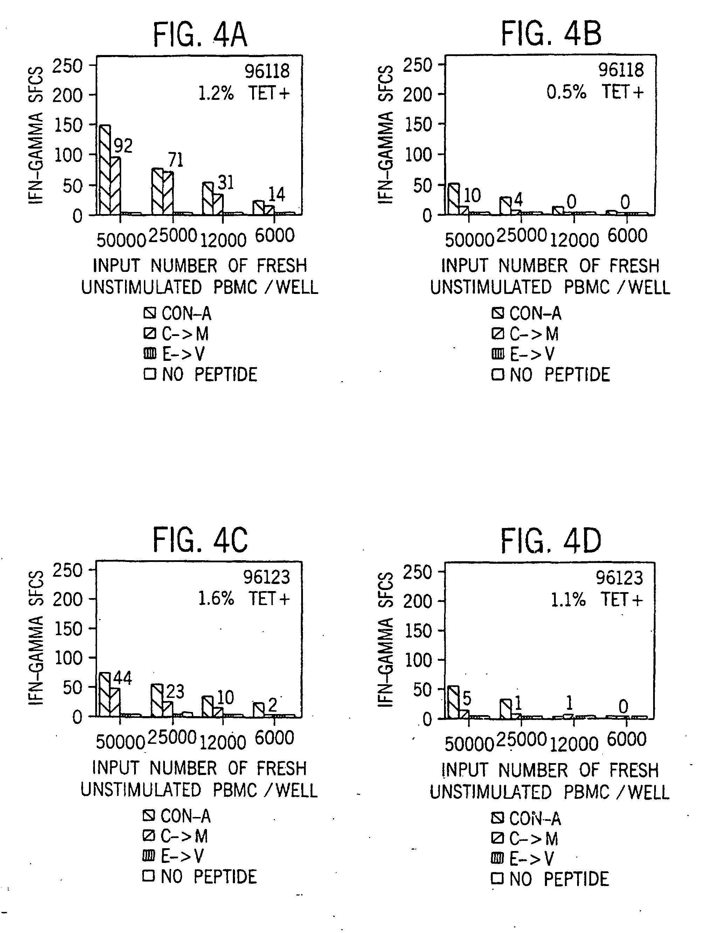

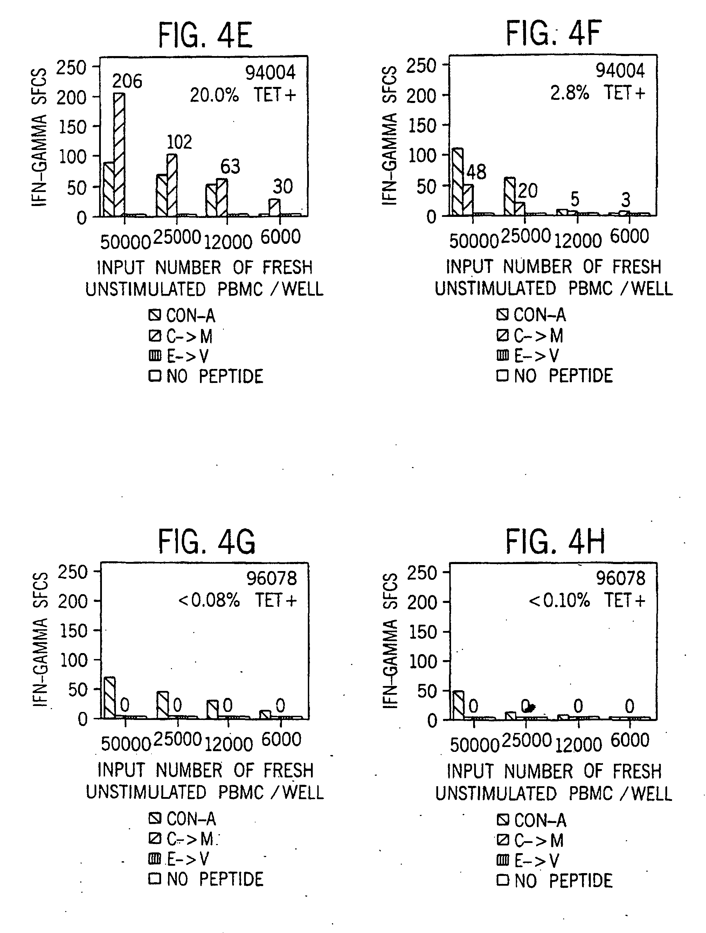

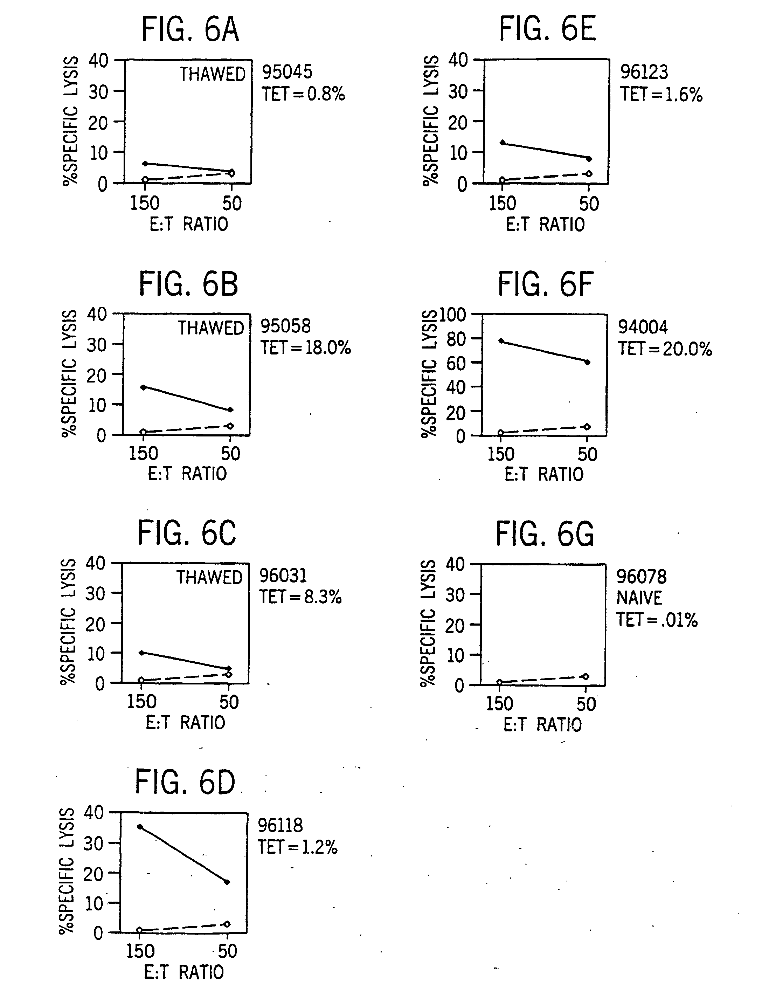

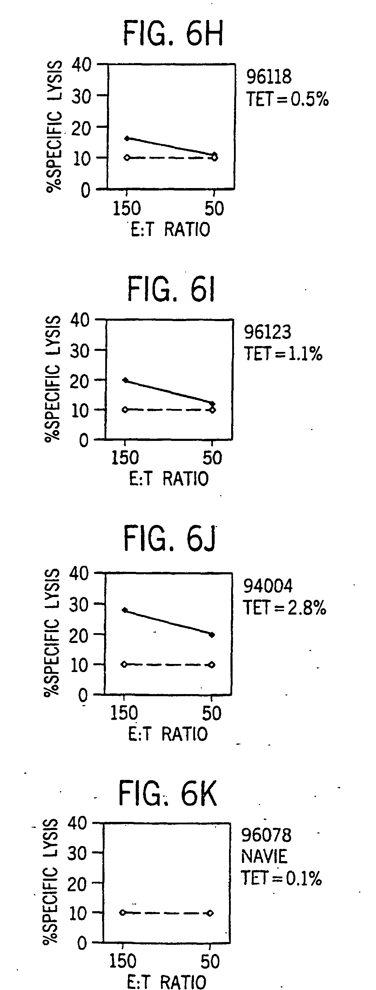

Another measure of the functionally active state of the induced CD8⁺ T lymphocytes is their lytic activity. While HIV- and SIV-specific CTL activity has been detected in fresh, unstimulated PBMC of seropositive humans (Pantaleo, et al., Nature 362:355-358, 1993; Walker, et al., Nature 328:345-348, 1987), and small intestine intraepithelial lymphocytes (iIEL) of chronically SIV-infected macaques (Couedel-Courteille, et al., J. Virol. 71:1052-1057, 1997), such responses have never been detected in the PBMC of SIV-infected macaques. In our study, detectable levels of fresh killing were present in five of six DNA/MVA vaccinated animals. In some cases these levels of specific lysis were as high as 75% at an E:T (effector:target) ratio of 150:1. While these experiments used unconventionally high E:T ratios, similar ratios were required to detect activity in the IEL of SIV-infected rhesus macaques (Couedel-Courteille, et al., J. Virol. 71:1052-1057, 1997), as well as in some HIV-infected patients ((Pantaleo, et al., Nature 362:355-358, 1993; Walker, et al., Nature 328:345-348, 1987). It is interesting that although animals 96118 and 96123 had very similar levels of tetramer staining, 1.2% and 1.6% respectively, animal 96118 had significantly higher levels of fresh killing, 36% versus 13% (Fig. 6). A similar difference was noted at this time point in ELISPOT assays in which animal 96118 had roughly twice as many p11C, C->M IFN-γ responding cells despite similar levels of tetramer staining (Fig. 4). Together these results suggest that there may be functional differences in the vaccine-induced CD8⁺ T cells in these two animals. Nonetheless, for the first time, the levels of CTL induced by a vaccine in non-human primates were high enough to induce detectable direct *ex vivo* cytotoxic activity.

While the role of CD8⁺ T cells in controlling HIV and SIV infections is well documented, and the induction of virus-specific CTL cell responses has been demonstrated by a variety of vaccines, the ability of vaccine-induced CD8⁺ T cell responses to control HIV and SIV infections remains elusive. Careful attention to the strength and breadth of a vaccine-induced CD8⁺ T cell response will be critical to the development of an effective HIV vaccine. The ability of this epitope-based DNA/MVA regimen to induce high levels of antigen-specific CTL against a single CTL epitope in non-human primates represents a first step toward addressing this issue.

The research that led to the present invention was undertaken primarily to develop a means for inducing a CTL immune response to HIV; however, the invention may be practiced using any viral CTL epitope capable of inducing a specific CTL response. Using the teachings disclosed herein, one of skill in the art could construct a live viral vaccine bearing a CTL epitope of interest using standard molecular biological techniques.

Although MVA was used as the live viral vaccine in the Examples, it should be appreciated that the present invention may be practiced using any suitable live viral vaccine, including, but not limited to, VEE (Venezuelan Equine Encephalitis virus), AAV (adeno-associated virus), and adenovirus.

Although the present research was designed to evaluate a vaccine regimen for protecting against viral infection or reducing viral load, it should also be appreciated that the present invention could be used for the manufacture of medicaments for cancer treatment because CTL are found to play a key role in tumor rejection. It is reasonably expected that an administration of DNA comprising a tumor epitope followed by a boost with MVA containing the same epitope would induce a CTL response.

The DNA-based vaccines may preferably be administered either through injection of a relatively large quantity of DNA into muscle tissues or of smaller quantities into the skin or mucosal surfaces. The latter method, which typically employs accelerated bombardment of tissue with microgram amounts of DNA-coated gold beads, has received considerable attention because the target tissue contains a relatively high concentration of professional antigen presenting cells (pAPC). The pAPC, especially dendritic cells, are believed to be critical to the induction of memory CTL responses.

The following nonlimiting examples are intended to be purely illustrative.

### EXAMPLES

### METHODS AND MATERIALS

### Animals

Rhesus macaques used in this study were identified as Mamu-A*01 positive by PCR-SSP and direct sequencing as previously described (Knapp, et al., Tissue Antigens 50:657-661,1997). The animals were maintained in accordance with the NIH Guide to the Care and Use of Laboratory Animals, and under the approval of the University of Wisconsin Research Animal Resource Center (RARC) review committee.

### DNA vectors pTH.HW and HC,C->M

The pTH.HW vector was derived from the pTH.H vector (Hanke, et al., Vaccine 16:426-435, 1998) which encodes a polyepitope of 20 HIV and 3 SIV CTL epitopes, including the Mamu-A*01-restricted CTL epitope TPYDINQML (SEQ ID NO:2) (Chen, et al., J. Immunol. 149:4060-4066, 1992). Following the construction of the pTH.H plasmid, the Mamu-A*01 CTL epitope was optimally defined and found to require an N-terminal cysteine residue (Allen, et al., J. Immunol. 160:6062-6071, 1998), now termed p11C, C->M (Kuroda, et al., J. Exper. Med. 187:1373-1381, 1998), having the sequence CTPYDINQM (SEQ ID NO:1). The pTH.H vector was modified to include the cysteine residue of the p11C, C->M epitope and was renamed pTH.HW (Hanke, et al., "Effective induction of SIV-specific CTL in macaques using a DNA prime-MVA boost vaccination regiment," J. Virol. *in press*)*.* The hepatitis B core antigen vector WRG7262 (PowderJect Vaccines Inc.) was also modified to. include the Mamu-A*01 CTL epitope CTPYDINQM (HC,C->M) within the immunodominant loop of the core protein between amino acids 80 and 81.

### DNA vaccinations

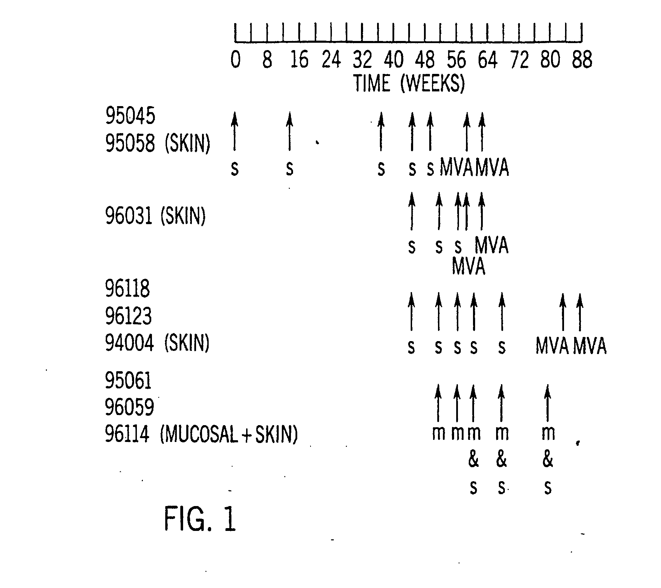

The DNA vaccinations were performed using the Dermal PowderJect XR ('gene gun') (PowderJect Vaccines, Madison, WI) as outlined in Table I and Fig. 1. Each immunization delivered 22 µg to 32 µg of DNA, precipitated on gold beads, unilaterally over a total of 8 skin sites in the abdominal or inguinal lymph node areas at 350 or 500 psi. Alternatively, mucosal tissues including tongue, buccal (cheek) and rectal tissues were targeted with 8 µg to 56 µg of DNA. Up to 12 abdominal sites, 4 tongue sites, 4 cheek sites, and 6 rectal sites were targeted depending on the size of each animal. The first vaccination delivered to each of the 9 animals employed only the pTH.HW plasmid, while all subsequent booster vaccinations involved co-delivery of equal molar amounts of both the pTH.HW and HC,C->M vectors.

### MVA Inoculations

Six of the DNA vaccinated rhesus macaques were inoculated with attenuated vaccinia virus Ankara (MVA) encoding the same HIV/SIV polyepitope HW (MVA.HW) used in the pTH.HW vector (Hanke, et al., Vaccine 16:439-445, 1998; Hanke, et al., J. Gen. Virol. 79:83-90, 1998; Hanke, et al., Vaccine 16:426-435, 1998). Each vaccination consisted of 5x10⁸ plaque forming units (PFU) of MVA.HW in a volume of 150 µl of PBS delivered intradermally and dispersed over three chest sites on each animal. 95045 and 95058 received their first MVA inoculation nine weeks after their last DNA vaccination, and 96031 two weeks after its last DNA vaccination (Fig. 1). The other three animals (94004, 96123 and 96118) received their MVA vaccination eighteen weeks after their last DNA vaccination. A second administration of MVA.HW was then given to each animal one month after receiving the first MVA inoculation. The MVA.HW was cultured on primary chick embryo fibroblasts (CEF) derived from eggs of a pathogen-free stock and prepared as described (Hanke, et al., Vaccine 16:439-445, 1998). No lesions were found associated with the inoculations.

### Isolation of PBMC

PBMC were isolated from EDTA-treated whole blood using Ficoll/diatrioate gradient centrifugation. Cells were washed twice in R10 media (RPMI 1640 supplemented with penicillin (50 U/ml), streptomycin (50 µg/ml), L-glutamine (2 mM), and 10% FBS (Biocell, Carson, CA).

### Generation of in vitro cultured CTL effector cells

PBMC were isolated as described above from blood drawn from the animals at two or four weeks after each DNA vaccination. Experiments employing PBMC were conducted using freshly isolated PBMC unless otherwise stated. CTL cultures were initiated by culturing 5x10⁶ PBMC with 10 µg of peptide in a final volume of 2 mls. On day three, a 1 ml aliquot of the medium was removed and replaced with R10 medium containing 20 U rIL-2 (recombinant interleukin 2)/ml (provided by M. Gately, Hoffman-La Roche, Nutley, NJ). Medium was supplemented every other day with rIL-2 until day 7, at which time the cells were stimulated with 5x10⁶ gamma-irradiated (3000 rad) autologous PBMC which had been incubated for 2 hours with the appropriate peptide. Following stimulation, rIL-2 was added every 2 days. On day 14, CTL activity of the cultures was assessed in a standard ⁵¹Cr-release assay. PBMC (peripheral blood mononuclear cells) were used as stimulators because it was noted that the B-LCL (B-lymphoblastoid cell line) from some animals grew less well than those from other animals, a factor which may affect the ability of B-LCL to equally stimulate CTL in a comparable manner. Peptides were obtained from Biosynthesis Inc. (Lexisville, TX) as desalted products. Lyophilized aliquots were resuspended in HBSS with 10% DMSO (Sigma) to a final concentration of 1 mg/ml.

### Cytotoxicity assays

CTL activity of *in vitro* stimulated CTL cultures was assessed as previously described (Allen, et al., J. Immunol. 160:6062-6071, 1998, which is incorporated by reference herein). Briefly, 5x10⁵ *Mamu*-*A***01*-transfected 721.221 cells or B-LCLs derived from a Mamu-A*01⁺ rhesus macaque were incubated for 1.5 hours with 80 µCi Na₂⁵¹CrO₄ (New England Nuclear Life Sciences Products) and 5 µg of corresponding peptide in 200 µl of R10 medium. These target cells were plated into duplicate wells of a 96-well U-bottom microtitre plate (5x10³ cells/well) and incubated with effector CTL for 5 hours. The reported percent specific lysis represents the ⁵¹Cr-release from CTPYDINQM peptide pulsed targets minus the ⁵¹Cr-release from target cells pulsed with an irrelevant SIV nef peptide (NQGQYMNTPR) (SEQ ID NO:3). Spontaneous release was always less than 25% of maximal release. Data reported for *in vitro* stimulated CTL cultures are based on single CTL assays tested at an effector to target (E:T) ratio of 20:1 unless otherwise noted. No appreciable difference in lysis was observed between either Mamu-A*01⁺ B-LCL or *Mamu*-*A***01*-721.221 cells as targets in ⁵¹Cr-release assays.

CTL activity of freshly isolated PBMC was assessed in a similar manner as for in vitro stimulated CTL cultures with the following exceptions: 1) target cells were Mamu-A*01⁺ B-LCLs, 2) triplicate rather than duplicate wells were plated, 3) the irrelevant peptide SNEGSYFF (SEQ ID NO:4) used in these experiments was derived from the influenza virus nucleoprotein (NP), and 4) data reported for fresh PBMCs were based on single CTL assays tested at effector to target ratios of 150:1 and 50:1.

### Mamu-A*01/CTPYDINOM tetramers

Soluble tetrameric Mamu-A*01 MHC class I/SIVgag CTPYDINQM peptide complexes were constructed as previously described (Altman, et al., Science 274:94-96, 1996, incorporated by reference herein) with the exception of the PCR primers required for amplification of the soluble MHC molecule. Primers Mamu-A*01-5p (SEQ ID NO:5) (5'-GGAATTCCATATGGGATCTCATTCAATGAAATATTTCTACA CCTCCAT G-3') and Mamu-A*01-3p (SEQ ID NO:6) (5'-CGCGGATCCGGACTGGGAAAACGGCTC-3') were designed to amplify the Mamu-A*01 heavy chain from a pKG5 vector encoding the rhesus MHC class I molecule Mamu-A*01. The PCR products were ligated to the *Nde I* and *BamH I* cloning sites of an expression vector (Altman, et al., Science 274:94-96, 1996). Expression of the rhesus MHC molecules was obtained and the MHC molecules were folded with human β2-microglobulin and peptide.

### Tetramer staining

Lymphocytes (2x10⁵) from two-week *in vitro* CTL cultures or 1x10⁶ fresh unstimulated PBMC were washed two times in FACS-buffer (PBS from Gibco with 2% FCS from BioCell) in a 96-well U-bottom plate. In a 100-pl volume, cells were stained in the dark for 30 minutes at room temperature with the Mamu-A*01-PE tetramer (0.5 µg/100 µl for *in vitro* cultures, 0.1 µg/100 µl for fresh PBMC) and an anti-rhesus CD3 FITC conjugated monoclonal antibody (10 µl; BioSource). To cool down the cells quickly to 4°C, the plates were placed at -20°C for 5 minutes. An anti-CD8a-PECy5 antibody (1 µl; Coulter) was then added for 10 minutes at 4°C, the cells were then washed three times with FACS-buffer and fixed with 450 µl of 2% paraformaldehyde (PFA). A p11C, C->M-specific CTL clone was also stained with 5 µl of isotype controls (mouse-IgG1-FITC, Sigma; mouse-IgG2a-PE, Sigma; and mouse-IgG1-PECy5, Coulter), anti-CD3-FITC, anti-CD8α-PECy5 (Coulter) and anti-CD8β-PE (Immunotech) to establish compensation parameters. Sample data were acquired on a Becton Dickinson FACSCalibur instrument and analyzed using CellQuest software (Becton Dickinson Immunocytometry Systems, San Jose, CA). Background tetramer staining of *in vitro* stimulated cultures from naive Mamu-A*01⁺ animals was routinely less than 0.2% and less than 0.08% for fresh, unstimulated PBMC (data not shown).

### IFN-γ ELISPOT Assay

96-well flat bottom plates (U-Cytech-BV, The Netherlands) were coated with 5 µg/well of anti-IFN-γ mAb MD-1 (U-Cytech-BV, The Netherlands) overnight at 4°C. The plates were washed 10 times with PBST (PBS containing 0.05% Tween-20) and blocked with PBSA (PBS containing 2% BSA) for 1 hour at 37°C. PBSA was discarded from the plates and freshly isolated PBMC resuspended in R5 media were added. The R5 also contained either 5 µg/ml Concanavalin-A (Con-A; Sigma Chemical, St. Louis, MO), 1 µg/ml of p11C, C->M peptide, 1 µg/ml of an irrelevant SIVenv peptide (ELGDYKLV; SEQ ID NO:7), or no peptide. 5 x 10⁴, 2.5 x 10⁴, 1.2 x 10⁴, and 6 x 10³ PBMC were plated in 100 µl/well, in triplicate wells and incubated overnight (14 hours) at 37°C, 5% CO₂. The cells were then removed and 200 µl/well of ice-cold deionized water was added to lyse the remaining PBMC. Plates were incubated on ice for 15 minutes and washed 20 times with PBST. Rabbit polyclonal biotinylated detector antibody (U-Cytech-BV, The Netherlands) was added at a concentration of 1 µg/well. The plates were incubated for 1 hour at 37°C, and washed 10 times with PBST. A gold-labeled anti-biotin IgG (U-Cytech BV, The Netherlands) was added (50 µl/well). The plates were incubated for 1 hour at 37°C and washed 10 times with PBST. Activator mix (U-Cytech BV, The Netherlands) was then added (30 µl/well) and developed for 30 minutes to allow a formation of silver salt precipitate at the site of gold clusters. The wells were then washed with distilled water, air dried, and the spots were counted (Klinman, et al., Curr. Prot. Immunol. 6.19:1-8, 1994).

### SIV-Infection of Macaques

Rhesus macaques were challenged intrarectally with 10 RMID (rhesus monkey infectious doses) of pathogenic SIVmac239.

### Viral Load Measurements

Two common tests were used to measure SIV RNA in the plasma of infected macaques. These tests are called the reverse transcriptase-polymerase chain reaction (PCR) test and the branched chain DNA (bDNA) test. RT-PCR is a more sensitive test and can detect much lower amounts of the virus than the bDNA test. The bDNA test can detect large numbers of the virus in the blood and is a more reproducable method, however, it will not detect the virus if only a low amount of the virus is present. These tests may be used to determine how much virus is in the body and how well the animals are responding to vaccine.

We preferentially used the bDNA test developed by Chiron Diagnostics. It is based on direct quantification of HIV-1 or SIV RNA and works by binding branched DNA probes to SIV RNA. This in turn binds enzymes which generates light. The amount of light is measured and is directly related to the amount of HIV-1 RNA in plasma. The bDNA technology can quantitate the major HIV subtypes found worldwide.

### Intracellular IFN-γ staining

1 x 10⁶ freshly isolated PBMC were incubated at 37°C for 1 hour with either 50 ng/ml PMA and 1 µg/ml Ionomycin, 5 µM of the p11C, CTPYDINQM peptide-pulsed Mamu-A*01-positive B-LCL, or Mamu-A*01-positive B-LCL alone as a control. Cells were treated with 10 µg/ml of Brefeldin A (BFA) for 4-5 hours at 37°C to inhibit export of protein from the endoplasmic reticulum. Cells were washed twice with FACS buffer (PBS + 2% FCS), stained with CD8a-PerCP (Becton Dickinson) and Mamu-A*01-PE tetramers as described above, fixed with PFA overnight, and washed twice with FACS-buffer. The cells were then treated with 150 µl of permeablization buffer (0.1% saponin in FACS-buffer) for 5 minutes at room temperature, washed once with 0.1% saponin, and incubated in the dark with 1 l of anti-human IFN-gamma-FITC mAb (Pharmingen) for 50 minutes. Cells were washed 3 times with 0.1% saponin-buffer and once with PBS before the 100 µl cell suspension was fixed with 450 µl of 2% paraformaldehyde (PFA).

### RESULTS

### Induction of CTL responses following a single skin DNA vaccination

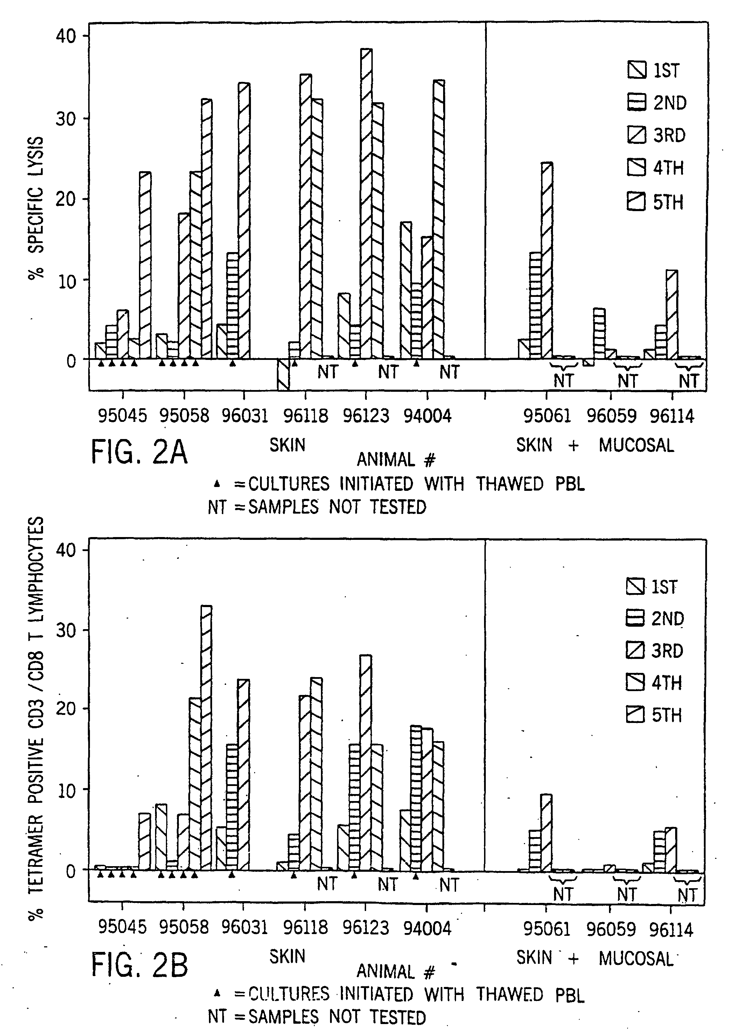

To determine whether a CTL response can be induced in primates using experimental vaccines encoding well characterized CTL epitopes, we used the PowderJect XR gene delivery system (PowderJect Vaccines Inc.) to vaccinate Mamu-A*01⁺ rhesus macaques with the pTH.HW vector encoding the Mamu-A*01-restricted SIV gag CTL epitope p11C, C->M (Allen, et al., J. Immunol. 160:6062-6071, 1998) (Fig. 1 and Table I). Two weeks following this first vaccination, PBMC were isolated, cultured for two weeks *in vitro* with the p11C, C->M peptide, and analyzed for CTL responses using standard ⁵¹Cr-release assays at an E:T of 20:1. Weak p11C, C->M-specific CTL responses (above levels of 6% detected in cultured PBMC from a naive Mamu-A*01⁺ macaque; data not shown) were detected in two of the six vaccinated animals from PBMC taken two weeks after the first vaccination (Fig. 2A), and in two additional animals from PBMC taken four weeks after the first vaccination (data not shown).

In parallel, tetrameric complexes were used to determine the percentage of CD3⁺/CD8α⁺ T lymphocytes in each culture that expressed T cell receptors specific for the p11C, C->M peptide/Mamu-A*01 complex. Staining of the two-week *in vitro* cultures with these tetramers revealed that four (Fig. 2C) of the animals were responding above background responses of 0.2% typically detected in cultured PBMC from a native Mamu-A*01⁺ macaque. Similar analysis of the four-week in vitro cultures revealed that all six animals had responded to the vaccination (data not shown). The relative levels of tetramer staining roughly reflected the relative percent specific lysis levels determined in the ⁵¹Cr-release assays of the same cultures. Therefore, while only low level antigen-specific responses were detected in these in vitro-stimulated cultures, p11C, C->M-specific CD8⁺ T cells could be detected in all six rhesus macaques following a single DNA vaccination.

### Booster DNA vaccinations employing co-delivery of the pTH, HW and HC,C->M vectors enhanced CD8⁺ T cell responses

Two to four booster vaccinations co-delivering the pTH.HW and an additional plasmid (HC,C->M) were then administered. The HC,C->M plasmid expresses a highly immunogenic hepatitis B core antigen (HBcAg) shown to augment the cellular immune responses to inserted T cell epitopes (Fomsgaard, et al., Scandinavian J. Immunol. 47:289-295, 1998; Ulrich, et al., Adv. Virus Res. 50:141-182, 1998; Schodel, et al., J. Biotechnology 44:91-96, 1996; Schodel, et al., Intervirology 39:104-110, 1996) and was used to stimulate T helper responses. PBMC were isolated two weeks after each DNA vaccination and in vitro stimulated cultures were initiated using fresh PBMC, with the exception of indicated time points where thawed PBMC were used. The administration of booster DNA vaccinations effectively increased responses in each of the six vaccinated macaques as measured by ⁵¹Cr-release assays (Fig. 2A) and tetramer staining (Fig. 2C). Following the third or fourth booster vaccination, levels of p11C, C->M-specific lysis in in vitro stimulated cultures were elevated to between 30% and 40%. Levels of tetramer positive CD3⁺/CD8α⁺ T lymphocytes were also increased to the range of 15%-35%. Therefore, booster DNA vaccinations effectively enhanced the levels of p11C, C->M-specific CD8⁺ T cell responses in all six vaccinated macaques. Following the fourth vaccination of some animals (96118, 96123, and 94004), the responses appeared to plateau. This may correlate with the reduced resting period between DNA vaccinations in this second group of three animals (Fuller, et al., Vaccine 15:924-926, 1997).

### DNA vaccinations targeted to mucosal tissues induced CTL responses in the periphery

Generation of a strong CTL response in the mucosa by vaccines will be important given the predilection of SIV for CD4⁺ lamina propria lymphocytes in the gut (Veazey, et al., Science 280:427-431, 1998). In order to assess the induction of CTL at mucosal sites an additional three Mamu-A*01⁺ rhesus macaques (95061, 96059, and 96114) were vaccinated at several mucosal sites including tongue, buccal (cheek) and rectal tissues. Although little or no response were detected in *in vitro* stimulated PBMC of these animals following the first vaccination with pTH.HW (Fig. 2B and 2D), two of the three animals demonstrated low but detectable p11C, C->M-specific responses by ⁵¹Cr-release assays and tetramer staining of *in vitro* stimulated cultures following the second vaccination (pTH.HW and HC,C->M) (Fig. 2B and 2D).

Subsequent vaccinations targeted to both mucosal and abdominal tissues continued to boost p11C, C->M-specific responses in two of the three animals (Fig. 2B and 2D). Although mucosal CD8⁺ T cell responses were not analyzed directly, the detection of responses in peripheral blood lymphocytes of these animals suggests that gene gun delivery of DNA to mucosal sites may have the potential to induce responses in the mucosa.

### DNA vaccinations induced detectable levels of tetramer positive cells in fresh, unstimulated PBMC

Due to the strong p11C, C->M-specific T cell responses observed in *in vitro* stimulated cultures, we reasoned that the frequency of p11C, C->M-specific CD8⁺ T lymphocytes might be high enough to detect low levels of tetramer positive cells in fresh, unstimulated PBMC. To follow the time course of antigen-specific CD8⁺ T lymphocyte induction in these vaccinated animals, fresh unstimulated PBMC were stained 2, 4, 6, and 13 days post vaccination. Levels of tetramer positive CD3⁺/CD8α⁺ T cells generally peaked 6 days after the DNA vaccination and declined by day 13 (data not shown). When the percentages of tetramer positive cells in fresh, unstimulated PBMC were followed in these six macaques over the last three vaccinations, three of the six animals showed levels of p11C, C->M-specific CD3⁺/CD8α⁺ T lymphocytes greater than 0.5% (Table 2). PBMC from animal 96059 never stained positive with the tetramer which was supported by the lack of response in *in vitro* stimulated cultures. However, the frequency of tetramer positive cells in fresh PBMC of animal 96123 was surprisingly low given that this animal had responded well in *in vitro* stimulated cultures. This DNA vaccine regimen, therefore, was able to induce levels of p11C, C->M-specific CD8⁺ T lymphocytes high enough to be detected in fresh, unstimulated PBMC using tetramers.

### MVA vaccination of DNA-primed macaques significantly boosted levels of epitope-specific CD8⁺ T lymphocytes in fresh, unstimulated PBMC

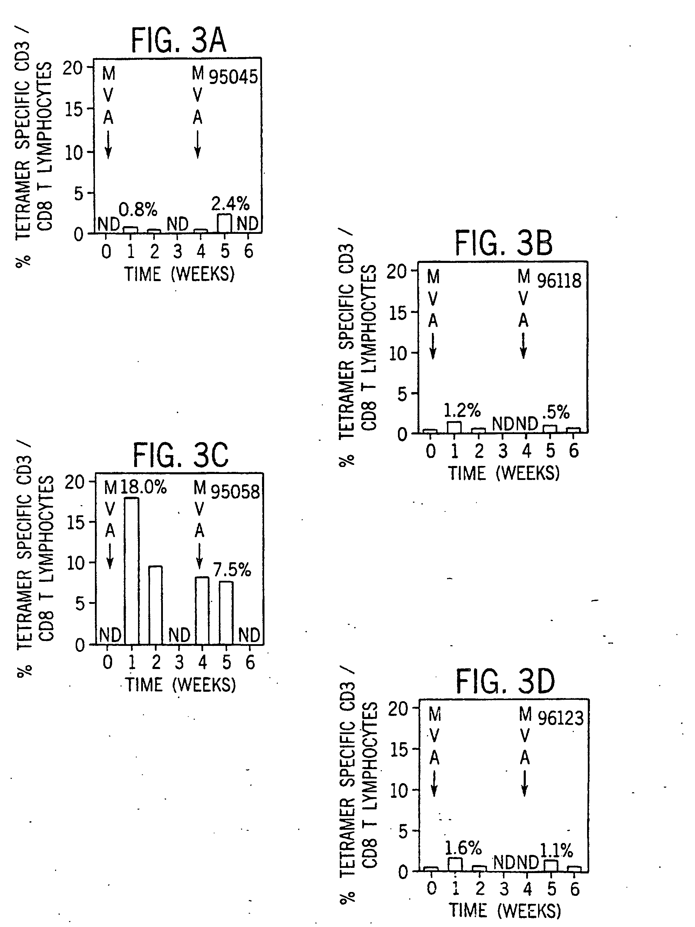

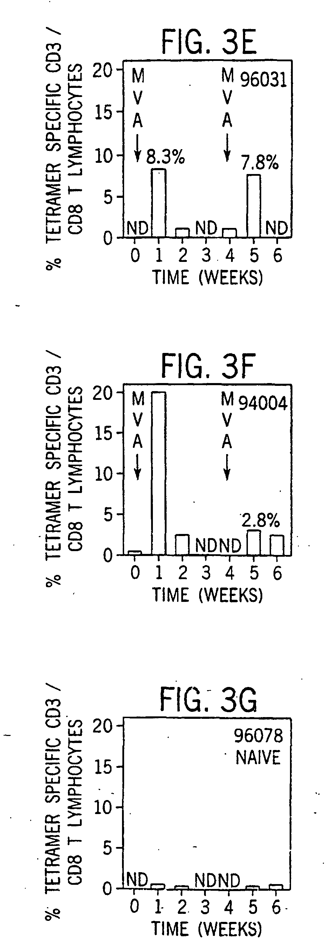

Because MVA was shown to be effective at both inducing and boosting CTL responses in mice and primates (Hanke, et al., Vaccine 16:439-445, 1998; Seth, et al., Proc. Natl. Acad. Sci. USA 95:10112-10115, 1998; Hanke, et al., "Effective induction of SIV-specific CTL in macaques using a DNA prime-MVA boost vaccination regiment," J. Virol. *in press*) we were interested in assessing the effects of MVA boosting DNA-vaccinated macaques. Fresh unstimulated, ficoll-separated PBMC isolated one to four weeks after the MVA were assessed for the percentage of CD3⁺/CD8α⁺ T lymphocytes which were specific for the p11C, C->M epitope. Tetramer analysis revealed that one week after the first MVA 0.8%, 18.0%, 8.3%, 1.2%, 1.6%, and 20.0% of the CD3⁺/CD8α⁺ T lymphocytes from these six macaques were specific for the p11C, C->M epitope (Fig. 3). These levels represented a significant increase over the 0.5% levels detected weeks earlier in some of the Mamu-A*01⁺ macaques after DNA vaccination (Table II), levels which had by this time likely declined to below 0.2%. Responses were then followed over the next four weeks and found to decline to levels below 1% in the majority of animals. Interestingly, in animals 95058 and 94004, which had demonstrated exceptionally high responses one week post MVA (18% and 20% respectively), levels were maintained above 8% and 2%, respectively, after two weeks. 95058 continued to maintain this high level out as far as four weeks (Fig. 3).

### A second administration of MVA boosted responses moderately

A second administration of MVA was given to all six macaques one month after receiving their first MVA. As before, responses peaked one week after this second MVA and increased over resting levels in the majority of animals (Fig. 3). Only in 95045, however, were stronger responses induced by this additional MVA than had been induced by the first MVA. In three of the macaques examined (96118, 96123, and 94004), responses again declined by two weeks post MVA. Therefore, while a second administration of MVA was capable of boosting levels of antigen-specific responses, the second MVA did not generally induce levels exceeding those detected following the first MVA. It should be noted, however, that the four week resting period between MVA inoculations may have been insufficient and that longer resting periods between MVA vaccinations, such as the twelve weeks used in other studies, may prove beneficial.

### Frequency of p11C, C->M-specific IFN-γ-producing T cells induced by vaccination

The ELISPOT assay represents an effective method for measuring the frequency of antigen-specific IFN-γ-producing T cells in the circulation (van der Meide, et al., J. Immunolog. Methods 144:203-213, 1991; Seth, et al., Proc. Natl. Acad. Sci. USA 95:10112-10116, 1998; Lalvani, et al., Clinical Science 95:531-538, 1998). Use of this assay demonstrated that each of the DNA/MVA vaccinated animals, 94004, 96118, 96123 mounted a strong antigen-specific response (Fig. 4). Fresh, unstimulated PBMC were tested at one and two weeks following administration of their first MVA with Con-A (positive control); the p11C, C->M peptide; an irrelevant envelope peptide (ELGDYKLV); or no peptide. One week following the first MVA, positive ELISPOT results were observed in each of the vaccinated animals. Animals 96118 and 96123 had 92 p11C, C->M-specific SFCs/50,000 input cells (effector frequency 1,842/10⁶ PBMC) and 44 p11C, C->M-specific SFCs/50,000 input cells (effector frequency 880/10⁶ PBMC), respectively (Fig. 4A). SFC responses in each of the animals titrated according to the number of input cells per well and were similar to values induced by Con-A stimulation. Animal 94004, with 20.0% tetramer positive cells, even demonstrated a better response to the p11C, C->M peptide, 206 SFCs/50,000 cells (effector frequency 4,132/10⁶ PBMC), than to Con-A (effector frequency 1,842/10⁶ PBMC). None of the animals demonstrated significant responses to the irrelevant peptide or to wells without any peptide even at 50,000 cells per well (less than 5 spots per well).

Samples derived two weeks following the first MVA were also analyzed (Fig. 4B). Although the levels of tetramer positive cells had declined slightly in 96118 and 96123, the number of IFN-γ producing cells detected in each of these animals at this two week time point were reduced to almost 10% of their previous values observed at one week. In 94004, the drop in tetramer positive cells by two weeks post MVA was reflected by a similar, although less dramatic drop in IFN-γ producing cells. Nonetheless, detectable levels of IFN-γ-producing cells were still present two weeks after administration of the MVA.

### Vaccine-induced tetramer positive lymphocytes from DNA/MVA vaccinated macaques produce IFN-γ in response to antigenic stimulation

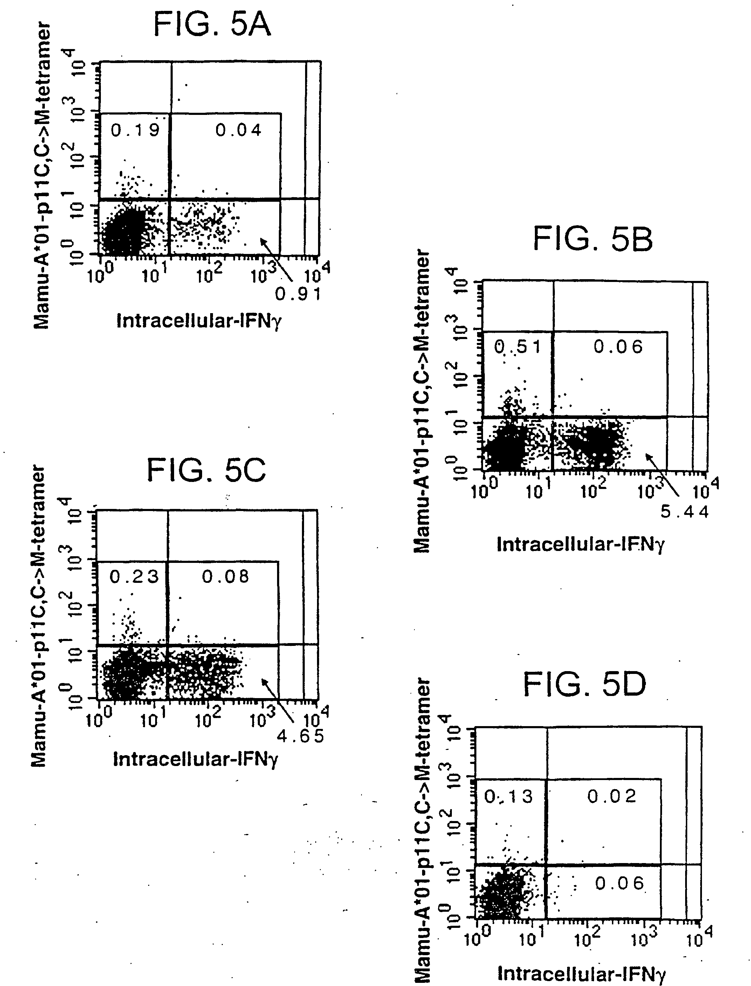

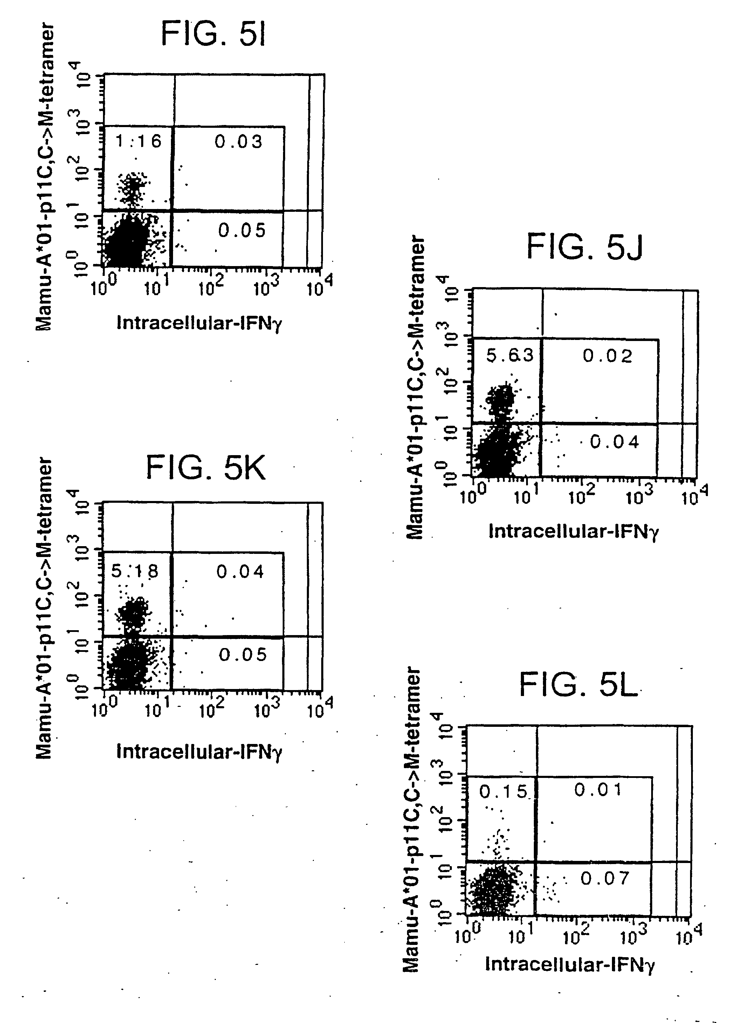

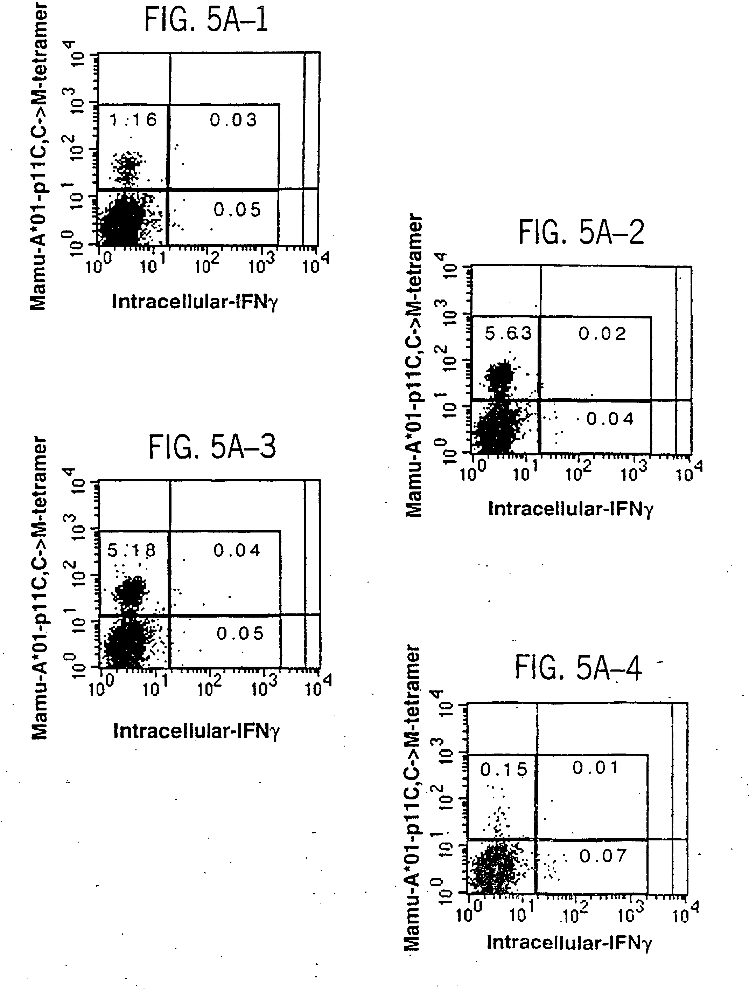

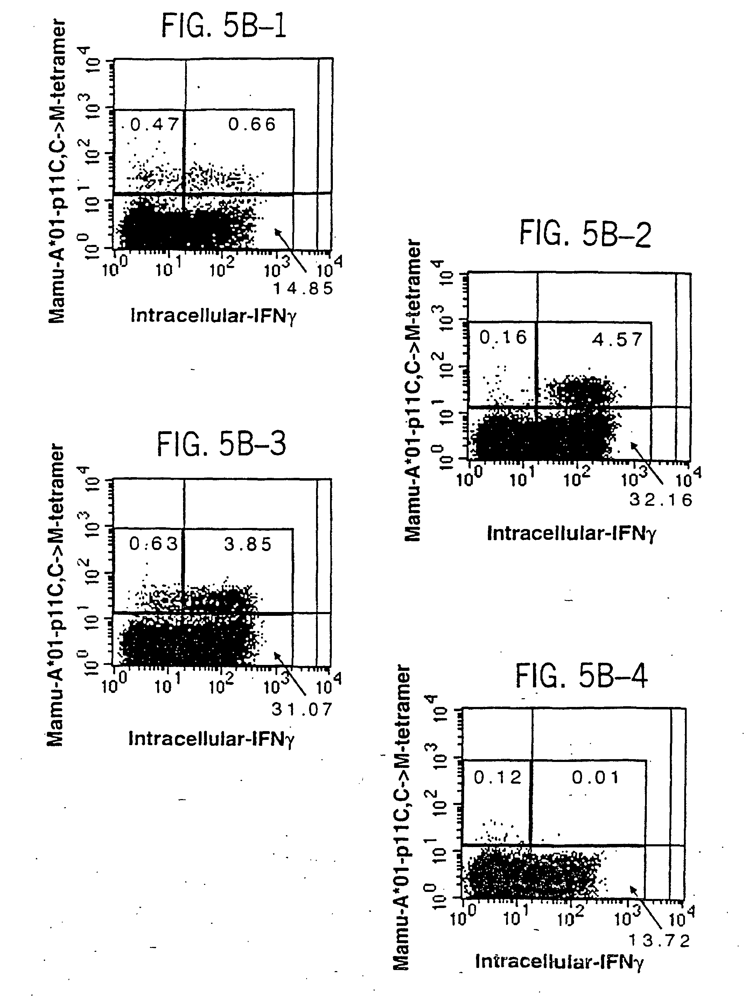

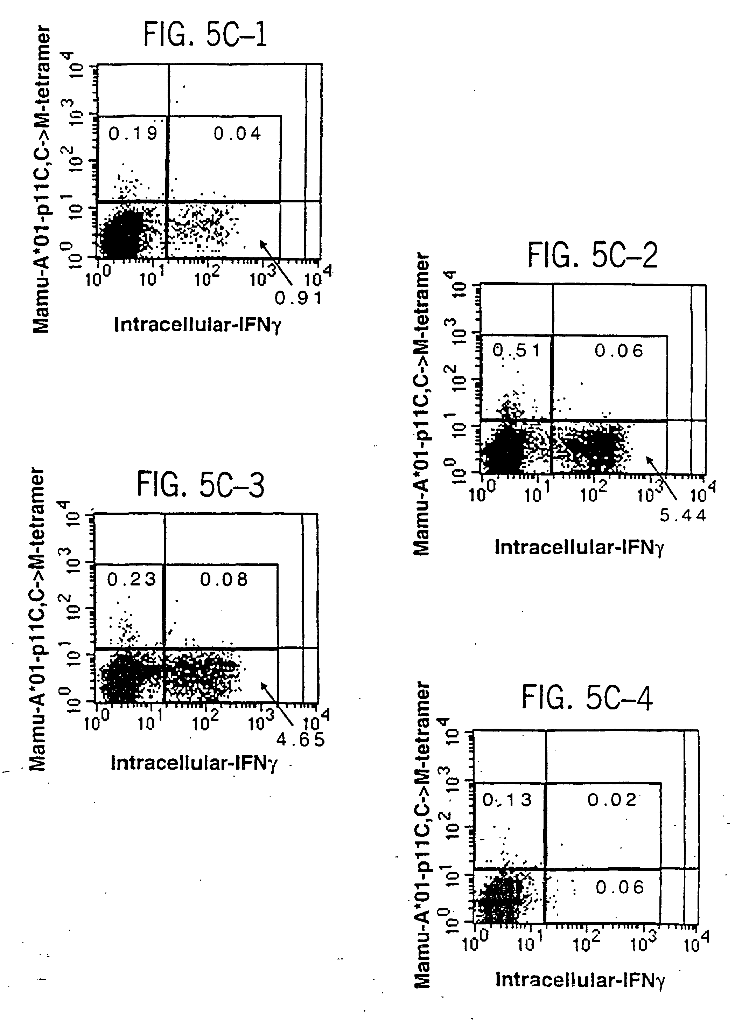

Given the surprisingly high levels of antigen-specific lymphocytes detected by the tetramer and ELISPOT assays, we used an additional assay to confirm these results. Initially, fresh PBMC from 95045, 95058, 96031 taken one week after the second MVA, and fresh PBMC from a naive Mamu-A*01⁺ animal 95084, were incubated with BFA (Brefeldin A) in the absence or presence of mitogenic or antigen-specific stimulation and stained for CD8α, Mamu-A*01/p11C, C->M tetramers, and IFN-γ. In the absence of stimulation no IFN-γ was produced (Fig. 5A). Treatment of cells with a mitogen such as PMA/Ionomycin induced the production of IFN-γ in tetramer-positive as well as tetramer-negative cells (Fig. 5B). Treatment of antigen-specific cells with the cognate peptide has been shown to internalize the T cell receptors (TCR) on the cell surface. Since the cells have been treated with BFA, egress of newly synthesized TCRs to the cell surface is prevented, abolishing tetramer staining. Treatment of PBMC from each of the vaccinated animals with the p11C, C->M peptide resulted in a reduction in the fraction of tetramer positive cells as expected (Fig. 5C). However, the fraction of cells which demonstrated the production of intracellular IFN-γ after p11C, C->M peptide stimulation was nearly equivalent to the fraction of untreated cells which were previously tetramer positive (Fig. 5A). These results indicate that the majority of tetramer positive CD8⁺ cells in these DNA/MVA vaccinated macaques are functionally active and capable of responding specifically to the p11C, C->M peptide by the production of intracellular IFN-γ. Treatment of fresh PBMC from these animals with an irrelevant peptide induced no internalization of TCR and little or no production of IFN-γ (data not shown).

### Direct ex-vivo cytotoxic activity detected in fresh PBMC from DNA/MVA vaccinated macaques

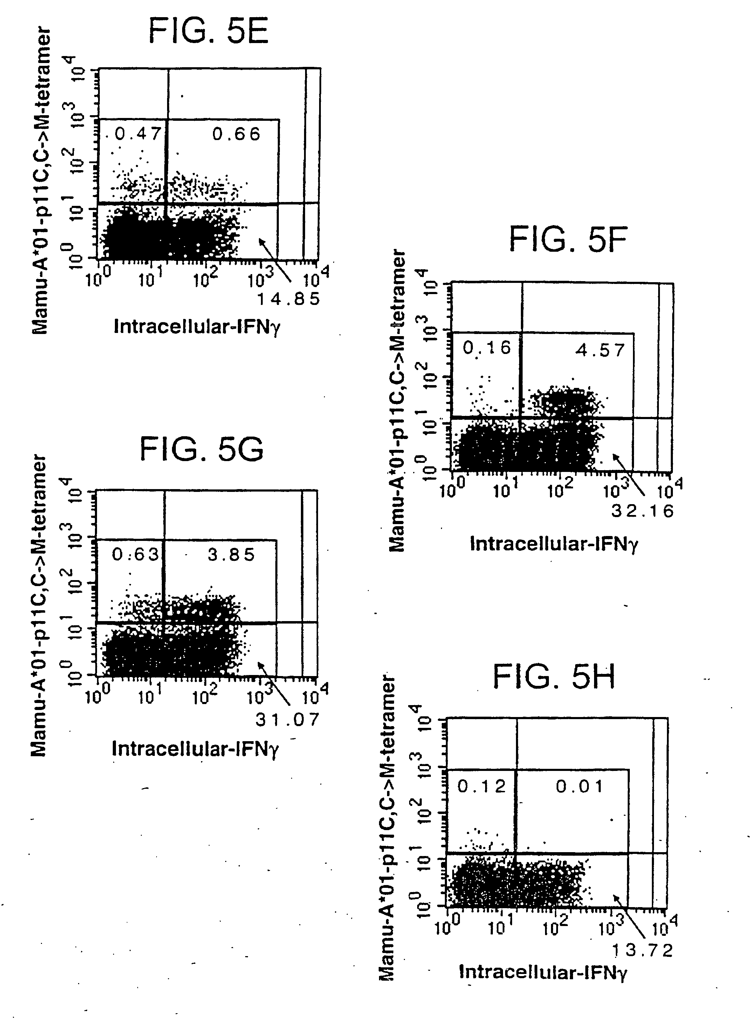

Since we induced high levels of peptide p11C, C->M-specific CD8⁺ T lymphocytes in our DNA/MVA vaccinated animals as detected by tetramer assays (Fig. 3), and those cells produced IFN-γ upon activation (Fig. 4 and 5), we were interested in determining whether we could detect direct *ex vivo* CTL activity against peptide-pulsed targets from fresh, unstimulated PBMC using ⁵¹Cr-release assays. PBMC were tested at E:T ratios of 150:1 and 50:1 against target B-LCLs pulsed with either peptide p11C, C->M or an irrelevant peptide. Normally, CTL activity in PBMC of vaccinated macaques, and even in chronically SIV infected animals, is only detectable after *in vitro* stimulation with peptide.

Thawed PBMC from animals 95045, 95058, and 96031 taken one week after their first MVA, and fresh PBMC from a naive Mamu-A*01⁺ macaque 96078, were initially tested. The percent specific lysis from thawed PBMC of 95045, and 96031 at the higher E:T ratio were very low, 5% and 10%, respectively (Fig. 6). 95058, however, which had 18.0% tetramer positive CD3⁺/CD8α⁺ T cells at this time point, demonstrated a more significant level of 15% specific lysis. These levels of specific lysis were repeatable in a second experiment using an additional aliquot of thawed cells from the same time point (data not shown).

Next we assessed for fresh killing in fresh, unstimulated PBMC from animals 96118, 96123, and 94004 one week after receiving their first MVA, at a point in which levels of tetramer staining were between 1.2% and 20.0%. Much more dramatic levels of fresh killing, between 13% and 77%, were detected in these animals than had been detected in thawed PBMC from 95045, 95058 and 96031.

Samples from animals 96118, 96123 and 94004 taken one week after the second MVA vaccination were then assessed. The levels of tetramer positive cells in these animals were not as high as levels detected one week after the first MVA. These lower levels of tetramer positive cells were paralleled by a significant reduction in the levels of specific lysis detected in each animal (Fig. 6). Nonetheless, significant responses were still detectable in 94004 (27% and 18%, respectively at E:T ratios of 150:1 and 50:1). Therefore, not only was this epitope-based DNA/MVA vaccine capable of inducing high levels of antigen-specific CD8⁺ T cells, the levels of induced CTL were high enough to exhibit cytolytic activity in fresh, unstimulated PBMC in the majority of animals.

The present invention is not limited to the exemplified embodiments, but is intended to encompass all such modifications and variations as come within the scope of the following claims.

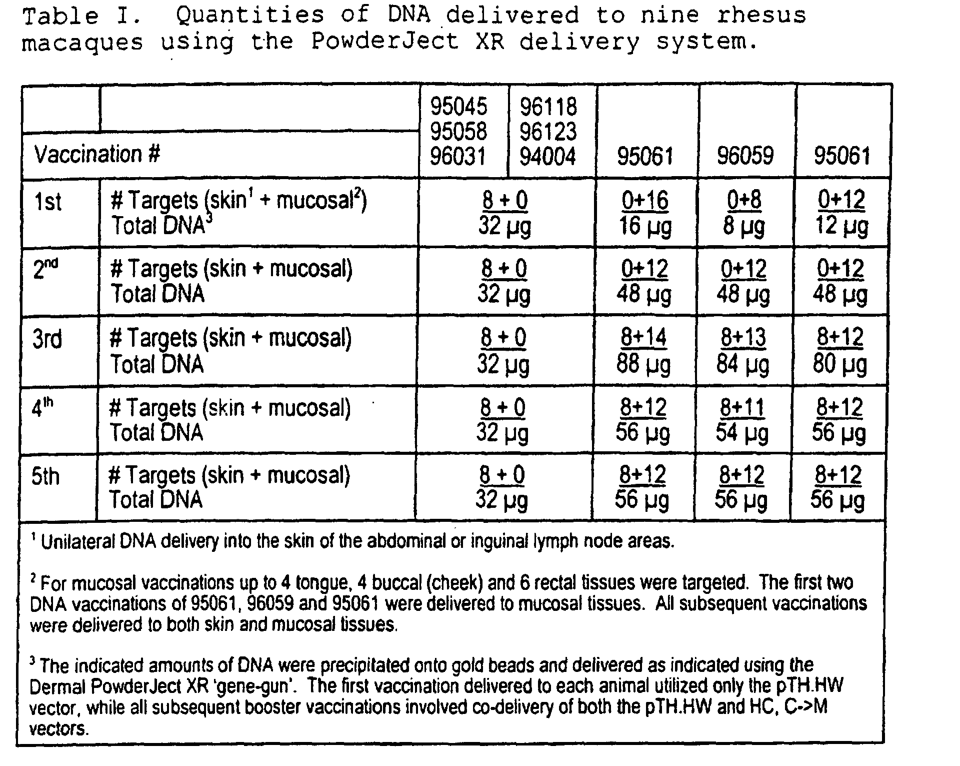

**Table II.**

| Percentage of tetramer positive CD3⁺/CD8a⁺ T lymphocytes detected in fresh, unstimulated PBMC from DNA vaccinated macaques. | | | | | |
|---|---|---|---|---|---|
| (# Vaccinations) Days post vaccination | (3) | (4) | | (5) | |
| | 14 days | 6 days | 13 days | 6 days | 13 days |
| 96118 | ND | ND | 0.23%¹ | 0.58% | 0.21% |
| 96123 | ND | ND | 0.10% | 0.07% | 0.10% |
| 94004 | ND | ND | 0.55% | 0.54% | 0.34% |
| 95061 | 0.23% | 0.65% | 0.16% | 0.43% | ND |
| 96059 | 0.03% | 0.01% | 0.07% | 0.03% | ND |
| 96114 | 0.15% | 0.11% | 0.21% | 0.38% | ND |
| ¹Tetramers typically stain <0.08% of fresh, unstimulated CD3⁺/CD8α⁺ T lymphocytes from PBL of naive Mamu-A*01⁺ animals. | | | | | |

## Claims

1. A combination comprising:
(a) a polynucleotide vaccine, comprising both (1) a polynucleotide sequence encoding a viral polyepitope and a major histocompatibility complex class I-restricted peptide epitope and (2) a polynucleotide sequence encoding a hepatitis B core antigen and a major histocompatibility complex class I-restricted peptide epitope, the encoding sequences being operably connected to a promoter functional in at least a portion of recipient cells, in an amount sufficient to induce in a primate a cytotoxic T lymphocyte response specific for the major histocompatibility complex class I-restricted peptide epitope; and
(b) a live virus vector comprising a polynucleotide coding sequence encoding a viral polyepitope and the major histocompatibility complex class I-restricted peptide epitope of (a) operably connected to a promoter functional in at least a portion of recipient cells, in an amount sufficient to boost the specific cytotoxic T lymphocyte response induced by the polynucleotide vaccine (a),
for use in a method of inducing an epitope-specific cytotoxic T lymphocyte immune response in a primate comprising the steps of delivering into cells of the primate the polynucleotide vaccine (a) and subsequently delivering the viral vector (b).

2. A combination according to claim 1, wherein the primate is a human.

3. A combination according to claim 1 or 2, wherein the major histocompatibility complex class I-restricted epitope is a viral epitope.

4. A combination according to claim 3, wherein the viral peptide epitope is a human immunodeficiency virus epitope.

5. A combination according to claim 4, wherein the viral epitope comprises a portion of the amino acid sequence of a polypeptide selected from the group consisting of human immunodeficiency virus gag, pol, nef, tat, rev, and env.

6. A combination according to any of the preceding claims, wherein delivery of the polynucleotide vaccine (a) is repeated at least once prior to delivery of the live virus vector (b).

7. A combination according to any of the preceding claims, wherein delivery of the live virus vector (b) is repeated at least once.

8. A combination according to any of the preceding claims, wherein the epitope-specific cytotoxic T lymphocyte response is detectable by tetramer staining of fresh, unstimulated polymorphonuclear blood cells.

9. A combination according to any of the preceding claims, wherein the epitope-specific cytotoxic T lymphocyte response induced by the live virus vector (b) is increased relative to the response induced by the polynucleotide vaccine (a).

10. A combination according to any one of claims 3 to 9, wherein, subsequent to delivery of the live virus vector (b), the primate is exposed to a virus comprising the viral epitope, and wherein the primate's viral load is lower than the viral load of a comparable naive primate similarly exposed to a virus bearing the viral epitope.

11. A combination according to any one of the preceding claims, wherein the polynucleotide vaccine (a) is suitable for delivery directly into the primate cells by particle bombardment.

12. A combination according to any one of claims 1 to 10, wherein the polynucleotide vaccine (a) is suitable for delivery into the primate by means of injection.

13. A combination according to any one of the preceding claims, wherein after delivery of the live virus vector (b) fresh polymorphonuclear blood cells from the primate produce gamma interferon following exposure to the major histocompatibility complex class I-restricted viral peptide of the polynucleotide vaccine (a).

14. A combination according to claim 1, wherein the major histocompatibility complex class I-restricted epitope is a tumor epitope.

15. Use of a polynucleotide vaccine (a) and a live viral vector (b) in the manufacture of a medicament for use in a method of inducing an epitope-specific cytotoxic T lymphocyte immune response in a primate comprising the steps of delivering the polynucleotide vaccine (a) into cells of the primate and subsequently delivering the live vaccine (b),
wherein the polynucleotide vaccine (a) comprises both (1) a polynucleotide sequence encoding a viral polyepitope and a major histocompatibility complex class I-restricted peptide epitope and (2) a polynucleotide sequence encoding a hepatitis B core antigen and a major histocompatibility complex class I-restricted peptide epitope, the encoding sequences being operably connected to a promoter functional in at least a portion of recipient cells, in an amount sufficient to induce in a primate a cytotoxic T lymphocyte response specific for the major histocompatibility complex class I-restricted peptide epitope; and
the live virus vector (b) comprises a polynucleotide coding sequence encoding a viral polyepitope and the major histocompatibility complex class I-restricted peptide epitope of (a) operably connected to a promoter functional in at least a portion of recipient cells, in an amount sufficient to boost the specific cytotoxic T lymphocyte response induced by the polynucleotide vaccine of (a).

16. Use of a polynucleotide vaccine, comprising: both (1) a polynucleotide sequence encoding a viral polyepitope and a major histocompatibility complex class I-restricted peptide epitope and (2) a polynucleotide sequence encoding encoding a hepatitis B core antigen and a major histocompatibility complex class I-restricted peptide epitope, the encoding sequences being operably connected to a promoter functional in at least a portion of recipient cells, in an amount sufficient to induce in a primate a cytotoxic T lymphocyte response specific for the major histocompatibility complex class I-restricted peptide epitope;
in the manufacture of a medicament for use in a method of inducing an epitope-specific cytotoxic T lymphocyte immune response in a primate comprising the steps of:
(a) delivering into cells of the primate the polynucleotide vaccine; and
(b) following step (a), delivering a live virus vector comprising a polynucleotide coding sequence encoding a viral polyepitope and the major histocompatibility complex class I-restricted peptide epitope of (a) operably connected to a promoter functional in at least a portion of recipient cells, in an amount sufficient to boost the specific cytotoxic T lymphocyte response induced by the polynucleotide vaccine of step (a).

17. Use of a live virus vector comprising a polynucleotide coding sequence encoding a viral polyepitope and a major histocompatibility complex class I-restricted peptide epitope operably connected to a promoter functional in at least a portion of recipient primate cells in the manufacture of a medicament for use in a method of inducing an epitope-specific cytotoxic T lymphocyte immune response in a primate comprising the steps of:
(a) delivering into cells of the primate a polynucleotide vaccine, the vaccine comprising both (1) a polynucleotide sequence encoding a viral polyepitope and a major histocompatibility complex class I-restricted peptide epitope and (2) a polynucleotide sequence encoding a hepatitis B core antigen and a major histocompatibility complex class I-restricted peptide epitope, the encoding sequences being operably connected to a promoter functional in at least a portion of recipient cells, and
(b) following step (a), delivering the live virus vector in an amount sufficient to boost the specific cytotoxic T lymphocyte response induced by the polynucleotide vaccine of step (a);
wherein the major histocompatability complex class I-restricted peptide epitope of the live virus vector is the same as the major histocompatability complex class-I-restricted peptide epitope of the polynucleotide vaccine.

## Patentansprüche

1. Kombination, umfassend:
a) Polynukleotidimpfstoff, umfassend sowohl (1) eine Polynukleotidsequenz, die ein virales Polyepitop und ein Haupthistokompatibilitätskomplex Klasse I-bezogenes Peptidepitop kodiert, als auch (2) eine Polynukleotidsequenz, die ein Hepatitis B-Kern-Antigen und ein Haupthistokompatibilitätskomplex Klasse I-bezogenes Peptidepitop kodiert, wobei die kodierenden Sequenzen operativ mit einem Promotor, der wenigstens in einem Teil der Empfängerzellen funktional ist, verbunden sind, in einer Menge, die ausreicht, um bei einem Primaten eine zytotoxische T-Lymphozytenantwort, die für das Haupthistokompatibilitätskomplex Klasse I-bezogene Peptidepitop spezifisch ist, zu induzieren; und
b) einen Lebendvirus-Vektor, umfassend eine Polynukleotid-kodierende Sequenz, welche ein virales Polyepitop und das Haupthistokompatibilitätskomplex Klasse I-bezogene Peptidepitop von (a) kodiert, die operativ mit einem Promotor, der wenigstens in einem Teil der Empfängerzellen funktional ist, verbunden ist, in einer Menge, die ausreicht, um die spezifische zytotoxische T-Lymphozytenantwort, die durch den Polynukleotidimpfstoff (a) induziert wurde, zu steigern,
zur Verwendung in einem Verfahren zum Induzieren einer Epitop-spezifischen zytotoxischen T-Lymphozytenimmunantwort in einem Primaten, umfassend die Schritte des Abgebens des Polynukleotidimpfstoffes (a) in Zellen des Primaten und nachfolgendes Abgeben des viralen Vektors (b).

2. Kombination gemäß Anspruch 1, wobei der Primat ein Mensch ist.

3. Kombination gemäß Anspruch 1 oder 2, wobei das Haupthistokompatibilitätskomplex Klasse I-bezogene Epitop ein virales Epitop ist.

4. Kombination gemäß Anspruch 3, wobei das virale Peptidepitop ein Epitop des menschlichen Immunschwächevirus ist.

5. Kombination gemäß Anspruch 4, wobei das virale Epitop einen Teil der Aminosäuresequenz eines Polypeptids, das aus der Gruppe, die aus dem menschlichen Immunschwächevirus gag, pol, nef, tat, rev und env besteht, ausgewählt wird, umfasst.

6. Kombination gemäß einem der vorhergehenden Ansprüche, wobei die Abgabe des Polynukleotidimpfstoffes (a) wenigstens einmal vor der Abgabe des Lebendvirus-Vektors (b) wiederholt wird.

7. Kombination gemäß einem der vorhergehenden Ansprüche, wobei die Abgabe des Lebendvirus-Vektors (b) wenigstens einmal wiederholt wird.

8. Kombination gemäß einem der vorhergehenden Ansprüche, wobei die Epitopspezifische zytotoxische T-Lymphozytenantwort durch Tetramerfärben von frischen, nichtstimulierten polymorphkernigen Blutzellen nachweisbar ist.

9. Kombination gemäß einem der vorhergehenden Ansprüche, wobei die Epitopspezifische zytotoxische T-Lymphozytenantwort, die durch den Lebendvirus-Vektor (b) induziert ist, relativ zu der Antwort, die durch den Polynukleotidimpfstoff (a) induziert ist, erhöht ist.

10. Kombination gemäß einem der Ansprüche 3 bis 9, wobei der Primat nach der Abgabe des Lebendvirus-Vektors (b) gegenüber einem Virus, das das virale Epitop umfasst, ausgesetzt wird und wobei die virale Belastung des Primaten niedriger ist als die virale Belastung eines vergleichbaren naiven Primaten, der in ähnlicher Weise gegenüber einem Virus, das das virale Epitop trägt, ausgesetzt wird.

11. Kombination gemäß einem der vorhergehenden Ansprüche, wobei der Polynukleotidimpfstoff (a) zur Abgabe direkt in die Primatenzellen durch Teilchenbeschuss geeignet ist.

12. Kombination gemäß einem der Ansprüche 1 bis 10, wobei der Polynukleotidimpfstoff (a) zur Abgabe in den Primaten mittels Injektion geeignet ist.

13. Kombination gemäß einem der vorhergehenden Ansprüche, wobei nach der Abgabe des Lebendvirus-Vektors (b) frische polymorphkernige Blutzellen des Primaten nach Aussetzung gegenüber dem Haupthistokompatibilitätskomplex Klasse I-bezogenen viralen Peptid des Polynukleotidimpfstoffes (a) Gammainterferon erzeugen.

14. Kombination gemäß Anspruch 1, wobei das Haupthistokompatibilitätskomplex Klasse I-bezogene Epitop ein Tumorepitop ist.

15. Verwendung eines Polynukleotidimpfstoffes (a) und eines Lebendvirus-Vektors (b) bei der Herstellung eines Medikaments zur Verwendung in einem Verfahren zum Induzieren einer Epitop-spezifischen zytotoxischen T-Lymphozytenimmunantwort in einem Primaten, umfassend die Schritte des Abgebens des Polynukleotidimpfstoffes (a) in Zellen des Primaten und nachfolgendes Abgeben des Lebendimpfstoffes (b),
wobei der Polynukleotidimpfstoff (a) sowohl (1) eine Polynukleotidsequenz, die ein virales Polyepitop und ein Haupthistokompatibilitätskomplex Klasse I-bezogenes Peptidepitop kodiert, als auch (2) eine Polynukleotidsequenz, die ein Hepatitis B-Kern-Antigen und ein Haupthistokompatibilitätskomplex Klasse I-bezogenes Peptidepitop kodiert, wobei die kodierenden Sequenzen operativ mit einem Promotor, der wenigstens in einem Teil der Empfängerzellen funktional ist, verbunden sind, in einer Menge, die ausreicht, um bei einem Primaten eine zytotoxische T-Lymphozytenantwort, die für das Haupthistokompatibilitätskomplex Klasse I-bezogene Peptidepitop spezifisch ist, zu induzieren, umfasst; und
der Lebendvirus-Vektor (b) eine Polynukleotid-kodierende Sequenz, welche ein virales Polyepitop und das Haupthistokompatibilitätskomplex Klasse I-bezogene Peptidepitop von (a) kodiert, das operativ mit einem Promotor, der wenigstens in einem Teil der Empfängerzellen funktional ist, verbunden ist, in einer Menge, die ausreicht, um die spezifische zytotoxische T-Lymphozytenantwort, die durch den Polynukleotidimpfstoff (a) induziert wurde, zu steigern, umfasst.

16. Verwendung eines Polynukleotidimpfstoffes, umfassend sowohl (1) eine Polynukleotidsequenz, die ein virales Polyepitop und ein Haupthistokompatibilitätskomplex Klasse I-bezogenes Peptidepitop kodiert, als auch (2) eine Polynukleotidsequenz, die ein Hepatitis B-Kern-Antigen und ein Haupthistokompatibilitätskomplex Klasse I-bezogenes Peptidepitop kodiert, wobei die kodierenden Sequenzen operativ mit einem Promotor, der wenigstens in einem Teil der Empfängerzellen funktional ist, verbunden sind, in einer Menge, die ausreicht, um bei einen Primaten eine zytotoxische T-Lymphozytenantwort, die für das Haupthistokompatibilitätskomplex Klasse I-bezogene Peptidepitop spezifisch ist, zu induzieren,
bei der Herstellung eines Medikaments zur Verwendung in einem Verfahren zum Induzieren einer Epitop-spezifischen zytotoxischen T-Lymphozytenimmunantwort in einem Primaten, umfassend die Schritte:
a) Abgeben des Polynukleotidimpfstoffes in Zellen des Primaten; und
b) nach Schritt (a) Abgeben des Lebendvirus-Vektors, umfassend eine Polynukleotid-kodierende Sequenz , die ein virales Polyepitop und das Haupthistokompatibilitätskomplex Klasse I-bezogene Peptidepitop von (a) kodiert, die operativ mit einem Promotor, der wenigstens in einem Teil der Empfängerzellen funktional ist, verbunden ist, in einer Menge, die ausreicht, um die spezifische zytotoxische T-Lymphozytenantwort, die durch den Polynukleotidimpfstoff des Schrittes (a) induziert wurde, zu steigern.

17. Verwendung eines Lebendvirus-Vektors, umfassend eine Polynukleotid-kodierende Sequenz, die ein virales Polyepitop und ein Haupthistokompatibilitätskomplex Klasse I-bezogenes Peptidepitop kodiert, die operativ mit einem Promotor, der wenigstens in einem Teil der Empfängerprimatenzellen funktional ist, verbunden ist, bei der Herstellung eines Medikaments zur Verwendung in einem Verfahren zum Induzieren einer epitop-spezifischen zytotoxischen T-Lymphozytenimmunantwort in einem Primaten, umfassend die Schritte:
a) Abgeben eines Polynukleotidimpfstoffes in Zellen des Primaten, wobei der Impfstoff sowohl (1) eine Polynukleotidsequenz, die ein virales Polyepitop und ein Haupthistokompatibilitätskomplex Klasse I-bezogenes Peptidepitop kodiert, als auch (2) eine Polynukleotidsequenz, die ein Hepatitis B-Kern-Antigen und ein Haupthistokompatibilitätskomplex Klasse I-bezogenes Peptidepitop kodiert, wobei die kodierenden Sequenzen operativ mit einem Promotor, der wenigstens in einem Teil der Empfängerzellen funktional ist, verbunden sind, umfasst; und
b) nach Schritt (a), Abgeben des Lebendvirus-Vektors in einer Menge, die ausreicht, um die spezifische-zytotoxische T-Lymphozytenantwort, die durch den Polynukleotidimpfstoff von Schritt (a) induziert wurde, zu steigern,
wobei das Haupthistokompatibilitätskomplex Klasse I-bezogene Peptidepitop des Lebendvirus-Vektors das gleiche ist wie das
Haupthistokompatibilitätskomplex Klasse I-bezogene Peptidepitop des Polynukleotidimpfstoffes.

## Revendications

1. Combinaison comprenant :
(a) un vaccin polynucléotidique, comprenant à la fois (1) une séquence polynucléotidique codant un polyépitope viral et un épitope peptidique restreint au complexe majeur d'histocompatibilité de classe I et (2) une séquence polynucléotidique codant un antigène de noyau de l'hépatite B et un épitope peptidique du complexe majeur d'histocompatibilité de classe I, les séquences codées étant assemblées de façon opérationnelle à un promoteur fonctionnel dans une partie au moins des cellules réceptrices, en une quantité suffisante pour induire chez un primate une réponse lymphocytaire de type T cytotoxique spécifique de l'épitope peptidique restreint au complexe majeur d'histocompatibilité dé classe I et
(b) un vecteur viral vivant comprenant une séquence codant un polynucléotide codant un polyépitope viral et un épitope peptidique restreint au complexe majeur d'histocompatibilité de classe I de (a) assemblé de façon opérationnelle à un promoteur fonctionnel dans une partie au moins des cellules réceptrices, en une quantité suffisante pour augmenter la réponse lymphocytaire de type T cytotoxique induite par le vaccin polynucléotidique (a),
pour son utilisation dans un procédé d'induction d'une réponse immunitaire à lymphocytes T cytotoxiques spécifiques d'un épitope dans un primate comprenant les étapes de délivrer dans les cellules de ce primate le vaccin polynucléotidique (a) et ensuite délivrer le vecteur viral (b).

2. Combinaison selon la revendication 1, dans laquelle le primate est un humain.

3. Combinaison selon la revendication 1 ou 2, dans laquelle l'épitope restreint au complexe majeur d'histocompatibilité de classe I est un épitope viral.

4. Combinaison selon la revendication 3, dans laquelle l'épitope peptidique viral est un épitope du virus de l'immunodéficience humaine.

5. Combinaison selon la revendication 4, dans laquelle l'épitope viral comprend une partie d'une séquence d'acides aminés d'un polypeptide choisi dans le groupe constitué de gag, pol, nef, tat, rev, et env du virus de l'immunodéficience humaine.

6. Combinaison selon l'une quelconque des revendications précédentes dans laquelle la délivrance du vaccin polynucléotidique (a) est répétée au moins une fois avant la délivrance du vecteur viral vivant (b).

7. Combinaison selon l'une quelconque des revendications précédentes, dans laquelle la délivrance du vecteur viral vivant (b) est répétée au moins une fois.

8. Combinaison selon l'une quelconque des revendications précédentes, dans laquelle la réponse lymphocytaire de type T cytotoxique spécifique de l'épitope est détectable par la coloration d'un tétramère de cellules sanguines fraîches non stimulées polynucléaires.

9. Combinaison selon l'une quelconque des revendications précédentes, dans laquelle la réponse lymphocytaire de type T cytotoxique spécifique de l'épitope induite par le vecteur viral vivant (b) est augmentée par rapport à la réponse induite par le vaccin polynucléotidique (a).

10. Combinaison selon l'une quelconque des revendications 3 à 9, dans laquelle, après la délivrance du vecteur viral vivant (b), le primate est exposé à un virus comprenant un épitope viral, dans laquelle la charge virale du primate est inférieure à la charge virale d'un primate naïf comparable exposé de façon similaire à un virus portant l'épitope viral.

11. Combinaison selon l'une quelconque des revendications précédentes, dans laquelle le vaccin polynucléotidique (a) est adapté à une délivrance directe dans les cellules du primate par un bombardement de particules.

12. Combinaison selon l'une quelconque des revendications 1 à 10, dans laquelle le vaccin polynucléotidique (a) est adapté à une délivrance chez un primate par injection.

13. Combinaison selon l'une quelconque des revendications précédentes, dans laquelle après la délivrance du vecteur viral vivant (b), les cellules sanguines fraîches polynucléaires provenant du primate produisent de l'interféron gamma après exposition au peptide viral restreint au complexe majeur d'histocompatibilité de classe I du vaccin polynucléotidique (a).

14. Combinaison selon la revendication 1, dans laquelle l'épitope restreint au complexe majeur d'histocompatibilité de classe I est un épitope tumoral.

15. Utilisation d'un vaccin polynucléotidique (a) et d'un vecteur viral vivant (b) dans la fabrication d'un médicament pour son utilisation dans un procédé d'induction d'une réponse immunitaire lymphocytaire de type T cytotoxique spécifique d'un épitope chez un primate comprenant les étapes de délivrer le vaccin polynucléotidique (a) dans les cellules du primate et ensuite de délivrer le vaccin vivant (b),
dans laquelle le vaccin polynucléotidique (a) comprend à la fois (1) une séquence polynucléotidique codant un polyépitope viral et un épitope peptidique restreint au complexe majeur d'histocompatibilité de classe I et (2) une séquence polynucléotidique codant un antigène de noyau de l'hépatite B et un épitope peptidique du complexe majeur d'histocompatibilité de classe I, les séquences codées étant assemblées de façon opérationnelle à un promoteur fonctionnel dans une partie au moins des cellules réceptrices, en une quantité suffisante pour induire chez un primate une réponse lymphocytaire de type T cytotoxique spécifique de l'épitope peptidique restreint au complexe majeur d'histocompatibilité de classe I et
un vecteur viral vivant (b) comprenant une séquence codant un polynucléotide codant un polyépitope viral et un épitope peptidique restreint au complexe majeur d'histocompatibilité de classe I de (a) assemblé de façon opérationnelle à un promoteur fonctionnel dans une partie au moins des cellules réceptrices, en une quantité suffisante pour augmenter la réponse lymphocytaire de type T cytotoxique induite par le d'induction d'une réponse immunitaire lymphocytaire de type T cytotoxique spécifique d'un épitope chez un primate comprenant les étapes de délivrer dans les cellules de ce primate le vaccin polynucléotidique (a) et ensuite délivrer le vecteur viral (b).

16. Utilisation d'un vaccin polynucléotidique comprenant à la fois (1) une séquence polynucléotidique dans un polyépitope viral et un épitope peptidique restreint au complexe majeur d'histocompatibilité de classe I et (2) une séquence polynucléotidique codant un antigène de noyau de l'hépatite B et un épitope peptidique restreint au complexe majeur d'histocompatibilité de classe I, les séquences codantes étant assemblées de façon opérationnelle à un promoteur fonctionnel dans une partie au moins des cellules réceptrices, en une quantité suffisante pour induire chez un primate une réponse lymphocytaire de type T cytotoxique spécifique de l'épitope peptidique restreint au complexe majeur d'histocompatibilité de classe I, dans la fabrication d'un médicament pour l'utilisation dans un procédé d'induction d'une réponse immunitaire lymphocytaire de type T cytotoxique spécifique d'un épitope chez un primate comprenant les étapes de (a) délivrer dans les cellules de ce primate le vaccin polynucléotidique et (b) à la suite de l'étape (a), délivrer un vecteur viral vivant comprenant un polynucléotide contenant une séquence codant un polyépitope viral et un épitope peptidique restreint au complexe majeur d'histocompatibilité de classe I de (a) assemblés de façon opérationnelle à un promoteur fonctionnel dans au moins une partie des cellules réceptrices, en quantité suffisante pour augmenter la réponse spécifique lymphocytaire de type T cytotoxique induite par le vaccin polynucléotidique de l'étape (a).

17. Utilisation d'un vecteur viral vivant comprenant un polynucléotide codant une séquence codant un polyépitope viral et un épitope peptidique restreint au complexe majeur d'histocompatibilité de classe I assemblés de façon opérationnelle à un promoteur fonctionnel dans au moins une partie des cellules réceptrices du primate dans la fabrication d'un médicament pour son utilisation dans un procédé d'induction d'un réponse immunitaire de type lymphocytaire T cytotoxique spécifique d'un épitope chez un primate, comprenant les étapes de
(a) délivrer dans les cellules du primate un vaccin polynucléotidique, le vaccin comprenant à la fois (1) une séquence polynucléotidique codant un polyépitope viral et un épitope peptidique restreint au complexe majeur d'histocompatibilité de classe I et (2) une séquence polynucléotidique codant un antigène de noyau de l'hépatite B et un épitope peptidique restreint au complexe majeur d'histocompatibilité de classe I, les séquences codantes étant assemblées de façon opérationnelle à un promoteur fonctionnel dans au moins une portion des cellules réceptrice et
(b) à la suite de l'étape (a), la délivrance d'un vecteur viral vivant en une quantité suffisante pour augmenter la réponse spécifique lymphocytaire de type T cytotoxique induite par le vaccin polynucléotidique de l'étape (a) ; dans lequel l'épitope peptidique restreint au complexe majeur d'histocompatibilité de classe I du vecteur viral vivant est le même que l'épitope peptidique retreint au complexe majeur d'histocompatibilité de classe I du vaccin polynucléotidique.
